(19) Europäisches Patentamt
European Patent Office
Office européen des brevets

(11) Veröffentlichungsnummer : **0 447 013 B1**

(12) # EUROPÄISCHE PATENTSCHRIFT

(45) Veröffentlichungstag der Patentschrift : **03.08.94 Patentblatt 94/31**

(51) Int. Cl.[5] : **C07C 311/13,** C07C 311/21, C07C 311/08, A61K 31/18

(21) Anmeldenummer : **91250069.1**

(22) Anmeldetag : **12.03.91**

(54) **Fluorbenzolsulfonamide.**

(30) Priorität : **12.03.90 DE 4008179**

(43) Veröffentlichungstag der Anmeldung : **18.09.91 Patentblatt 91/38**

(45) Bekanntmachung des Hinweises auf die Patenterteilung : **03.08.94 Patentblatt 94/31**

(84) Benannte Vertragsstaaten : **AT BE CH DE DK ES FR GB GR IT LI LU NL SE**

(56) Entgegenhaltungen : **EP-A- 0 368 429**

(73) Patentinhaber : **SCHERING AKTIENGESELLSCHAFT**
**D-13342 Berlin (DE)**

(72) Erfinder : **Gries, Heinz, Dr.**
**Helmstedter Strasse 19**
**W-1000 Berlin 31 (DE)**
Erfinder : **Niedballa, Ulrich, Dr.**
**Gosslerstrasse 28A**
**W-1000 Berlin 33 (DE)**
Erfinder : **Weinmann, Hanns-Joachim, Dr.**
**Westhofener Weg 23**
**W-1000 Berlin 38 (DE)**
Erfinder : **Bauer, Hans, Dr.**
**Wendelsteinweg 24**
**W-1000 Berlin 42 (DE)**

**EP 0 447 013 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft den in den Patentansprüchen gekennzeichneten Gegenstand, das heißt Fluorbenzolsulfonamide, ihre Verwendung als NMR-Diagnostika, diagnostische Mittel, die diese Fluorbenzolsulfonamide enthalten sowie Verfahren zur Herstellung dieser Verbindungen und Mittel.

Die moderne Medizintechnik ermöglicht es, kleinste morphologische Strukturen mit einer Auflösung darzustellen, die der von Gewebeschnitten aus Anatomielehrbüchern nahekommt.

Es gelingt jedoch auch mit Hilfe der Ultraschall-, der Röntgen-Diagnostik, der Nuklearmedizin und selbst der Kernspintomographie nicht, Informationen über den stoffwechselphysiologischen Zustand eines Gewebes des lebenden Organismus zu erhalten. Für eine genauere Diagnose und insbesondere für eine Planung und Verlaufskontrolle eines therapeutischen Einsatzes ist jedoch diese Kenntnis von erheblicher Bedeutung, da eine optimale Therapie nur dann erfolgreich sein kann, wenn frühzeitig eine Aussage über deren Wirkung möglich ist.

Ein wichtiger Parameter der stoffwechselphysiologischen Aktivität ist der pH-Wert. Viele pathologische Prozesse haben eine Veränderung der Wasserstoffionen-Konzentration zur Folge. Eines der bekanntesten Beispiele ist die Freisetzung von Milchsäure infolge ungenügender Sauerstoffversorgung und dadurch bedingte anaerober Verstoffwechselung von Glucose. Eine anaerobe Glykolyse findet praktisch überall dort statt, wo eine ausreichende Versorgung mit Sauerstoff nicht mehr gewährleistet ist. Eine kurzfristige Ansäuerung ist zum Beispiel in Bereichen höchster Muskelaktivität festzustellen. Hier wird jedoch die anfallende Milchsäure in der Ruhepause relativ rasch abtransportiert, so daß im ruhenden Muskel keine Übersäuerung festzustellen ist. Anders sieht es jedoch in Bereichen permanenter Sauerstoffschuld aus. In ischaemischen Arealen (Infarkt) kommt es aufgrund der gesteigerten anaeroben Glykolyse zu einer Verschiebung des pH-Wertes. Ähnliche Effekte sind in schnell wachsenden Neoplasmen zu beobachten. Neben einer Regulationsstörung liegt im Bereich eines Tumors ein Sauerstoffmangel vor, so daß auch hier durch anaerobe Verstoffwechselung von Kohlenhydraten eine Ansäuerung auftritt.

Die Bestimmung des Gewebe-pH-Werts führt somit zu wichtigen Aussagen über Funktion, Zustand und Wachstum der Zellen, so daß es z.B. wünschenswert ist, metabolische Azidosen zu lokalisieren. (Am. J. Physiol. 246, R 409, 1984; R. Nuccitelli, D.W. Deamer, Eds. 1982 Intracellular pH: Its Measurement, Regulation and Utilization in Cellular Functions, Liss. New York).
Neben der Messung des pH-Wertes mit pH-Elektroden wurde neuerdings auch die NMR-Spektroskopie zu diesem Zweck eingesetzt. Mit deren Hilfe ist es erstmals gelungen, den PH-Wert des Gewebes ohne äußere Beeinflussung zu bestimmen.

Die Bestimmung des PH-Wertes mit Hilfe der NMR-Spektroskopie beruht auf der Messung der Signale einer chemischen Verbindung, die sich in einem pH-abhängigen, reversiblen Gleichgewicht befindet. Ist dieses Gleichgewicht langsam bezüglich der NMR-Zeitskala, so können die Signale sämtlicher Komponenten erhalten werden und die Signalintensitäten entsprechen den Konzentrationen der Gleichgewichtskomponenten. Bei einem schnellen Gleichgewicht ist dagegen nur ein Signal meßbar und die chemische Verschiebung ist durch die chemische Verschiebung der Gleichgewichtskomponenten und deren Konzentration gegeben.

In einem Zweikomponenten-Gleichgewicht kann dann, bei Kenntnis des $pK_a$-Wertes und der chemischen Verschiebung der Komponenten, der PH-Wert mit Hilfe der Henderson-Hasselbalch-Gleichung berechnet werden.

Aus der folgenden Tabelle ist ersichtlich, welche Atomkerne prinzipiell für die NMR-Bildgebung bzw. -Spektroskopie in Frage kommen:

| Kern | Frequenz bei 1 Tesla MHz | rel. Meßempfindlichkeit $^1H$ = 1 | Konzentration in biol. Gewebe | Chemical Shift | chemische Modifikationsmöglichkeit | $T_1$-Relaxationszeiten |
|---|---|---|---|---|---|---|
| $^1H$ | 42.6 | 1.0 | 100 mol/l | klein | sehr hoch | 0.1-3 sec |
| $^{19}F$ | 40.1 | 0.8 | << 1 mmol/l | sehr groß | sehr hoch | 1-5 sec |
| $^{23}Na$ | 11.3 | 0.09 | 100 mmol/l | - | praktisch null | < 0.1 sec |
| $^{31}P$ | 17.2 | 0.06 | 10 mmol/l | mittel | begrenzt | 1-5 sec |
| $^{13}C$ | 10.7 | 0.0002 | 1 mmol/l | sehr groß | sehr hoch | 1-10 sec |

Seit 15 Jahren wird der $^{31}P$-Kern als nicht-invasive Meßsonde für die intracelluläre pH-Wert-Messung eingesetzt (J. Biol. Chem. 248, 7276, 1973). Das pH-empfindliche Signal ist hierbei das Signal des anorganischen Phosphats aus dem Gleichgewicht Hydrogenphosphat-Dihydrogenphosphat; als Referenz dient das $^{31}P$-Signal des Phosphokreatins.

Die Verwendung des $^{31}P$-Kerns für die pH-Wert-Bestimmung hat jedoch auch ihre Grenzen: So ist eine exakte Bestimmung des pH-Wertes in einem gut lokalisierten Gewebevolumen beim Menschen selbst mit dem Einsatz von 2T Kernspintomographen nicht möglich. Dies liegt sowohl an den relativ niedrigen Phosphatkonzentrationen als auch an der Tatsache, daß das $^{31}P$-Signal meßtechnisch schwierig zu erfassen ist. Störsignale im Bereich des anorganischen Phosphats, Überlagerung des anorganischen P-Signals durch andere P-Metabolite oder das Fehlen eines Referenzsignals können eine pH-Messung verhindern. Weitere Schwierigkeiten liegen in der geringen Empfindlichkeit des Kerns und der geringen pH-Abhängigkeit der chemischen Verschiebung. Die Genauigkeit der pH-Messung wird vor allem durch die Bestimmung der chemischen Verschiebungen der Signale beeinflußt und ist nicht besser als 0,2 pH. Außerdem können Resonanzsignale bei der Verwendung endogener Phosphate gänzlich fehlen, weil die Verbindungen sich nur in so geringen Konzentrationen (z.B. im Darm oder in Ehrlich Aszites-Tumorzellen) anreichern, daß eine pH-Wert Bestimmung nicht möglich ist.

Aufgrund dieser Gegebenheiten ist nur eine recht ungenaue pH-Bestimmung in vergleichsweise großen Volumina möglich. Für die Erstellung eines befriedigenden $^{31}P$-Spektrums werden bei einer Akkumulationszeit von 15 Minuten Signale aus dem Meßvolumen von etwa 100 ccm aufgenommen.

Bei der Nutzung anderer Kerne als $^{31}P$ ist der $^{19}$Fluor-Kern der Kern der Wahl, da er ein leicht meßbares NMR-Signal, das dem des Wasserstoffprotons sehr ähnlich ist (er besitzt ebenso wie $^1H$ einen Kernspin von 1/2), liefert, d.h. es können die gleichen Empfänger- und Sender-Spulen wie in der $^1H$-NMR-Diagnostik verwendet werden, eine hohe Empfindlichkeit (ca. 83% von $^1H$) besitzt, in 100 % Häufigkeit vorliegt und die Signale über einen großen Frequenzbereich verteilt sind. Als weitere Vorteile sind die Abwesenheit von Fluor im Organismus (mit Ausnahme der Zähne), so daß keine Komplikationen mit endogen F-Signalen auftreten können (Fehlen eines $^{19}F$-Background-Signals) sowie die günstige chemische Zugänglichkeit anzuführen.

Informationen, die über die Verwendung von F-Molekülen in der NMR-Diagnostik erhältlich sind, können durch kein anderes diagnostisches bildgebendes bzw. quasi-bildgebendes Verfahren erzielt werden: das Signal kann sich im Körper - je nach chemischem Zustand - stark verändern, erlaubt somit die Quantifizierung biochemischer Reaktionen und ermöglicht eine direkte Beobachtung physiologischer Vorgänge. Trotz dieser verführerischen Eigenschaften muß auf die problematische Konzentration hingewiesen werden. Ein sinnvolles Experiment bedarf $^{19}F$-Konzentrationen von > 1 mmol F/l, d.h. die zu applizierenden Verbindungen müssen eine hervorragende Verträglichkeit aufweisen und eine gute Löslichkeit in Wasser besitzen, damit durch Verwendung von Lösungen hoher Konzentrationen möglichst kleine Volumina verwendet werden können.

Die Frequenz (chemical shift) einer Fluorlinie wird durch die Lage des F-Atoms im Molekül bestimmt. Prinzipiell gilt dies für alle anderen Atomkerne auch, jedoch ist im Falle des Fluoratoms der chemical shift besonders stark ausgeprägt. Um eine Verschiebung des Fluorsignals zu beobachten bzw. zu quantifizieren bedarf es einer Bezugs(Referenz)-Linie. Diese Frequenzlinie kann das $^1H$Signal, ein externer F-Standard bzw. eine

sich nicht verändernde F-Linie, die sich ebenfalls in dem zu vermessenden Areal befindet, sein. Diese Referenzlinie kann sich in einem anderen, ähnlich verteilenden Molekül oder vorzugsweise in dem als Indikator benutzten Molekül selbst befinden. Die günstigste Situation liegt im letztgenannten Fall vor, da hierbei nur eine Substanz appliziert wird und keinerlei Probleme mit Suszeptibilitätseffekten auftreten, so daß eine zweifelsfreie Zuordnung der Signale möglich ist.

Es besteht daher ein Bedarf, geeignete Verbindungen zu finden, die auf eine Veränderung des pH-Werts mit einer veränderten Meßgröße (Resonanzfrequenz) im NMR-Spektrum bei gleichzeitigem Vorhandensein einer Referenzlinie reagieren. Weiterhin müssen diese Verbindungen bzw. die diese Verbindungen enthaltenden diagnostischen Mittel folgende Eigenschaften aufweisen:

a) eine große chemische Verschiebung pro pH-Einheit;
b) geeignete pK-Werte für in-vivo-Messungen;
c) eine für die Diagnostik geeignete Pharmakokinetik;
d) eine für eine Messung genügend hohe Anreicherung in den Zielorganen;
e) gute Verträglichkeit und geringe Toxizität;
f) metabolische Stabilität;
g) hohe chemische Stabilität und Lagerfähigkeit,
h) gute Wasserlöslichkeit.

Die bisher (und nur für in-vitro-Untersuchungen!) beschriebenen Verbindungen (Annals of the New York Academy of Science, S.M. Cohen, Ed. 1987, 508 33) erfüllen diese Voraussetzungen nicht. So ist mit ihnen z.B. eine genauere pH-Bestimmung als mit $^{31}P$ nicht möglich, da die pH-Abhängigkeit der chemischen Verschiebung zu gering ist (≤ 1 ppm/pH) und/oder ihre pH-Werte liegen außerhalb des physiologischen Bereichs und/oder ihre Resonanzfrequenzen sind nicht nur vom pH sondern auch von der Feldstärke abhängig. Auch sind die beschriebenen Verbindungen auf Grund ihrer schlechten Verträglichkeit für einen tierexperimentellen oder gar klinischen Einsatz nicht geeignet.

Der Erfindung liegt somit die Aufgabe zugrunde, Verbindungen und Mittel, die die oben genannten Eigenschaften aufweisen, zur Verfügung zu stellen sowie Verfahren zu ihrer Herstellung zu schaffen. Diese Aufgabe wird durch die Erfindung gelöst.

Es wurde gefunden, daß sich Fluorbenzolsulfonamide der allgemeinen Formel I

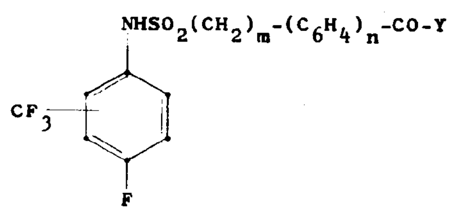

(I),

worin

m für die Ziffern 0, 1, 2, 3 oder 4,
n für die Ziffern 0 oder 1 und
Y für den Rest einer Aminocarbon- oder Aminosulfonsäure

stehen mit der Maßgabe, daß m und n nicht gleichzeitig für die Ziffer 0 stehen sollen und gewünschtenfalls die Säuregruppen in Form ihrer Amide oder in Form von Salzen mit organischen oder anorganischen Basen vorliegen,
überraschenderweise hervorragend zur Herstellung von NMR-Diagnostika eignen.

Enthalten die erfindungsgemäßen Verbindungen mehr als eine Säuregruppe im Molekül, so können sowohl alle (vgl. Beispiel 10) als auch nur eine (vgl. Beispiel 55) der Säuregruppen in Form ihrer Amide oder Salze vorliegen.

Als Aminocarbon- und Aminosulfonsäuren (bzw. deren Amide) sind sowohl deren natürliche als auch synthetische Vertreter geeignet. Beispielhaft genannte Reste Y seien

$$-NH-CH_2COR^1$$

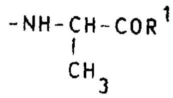

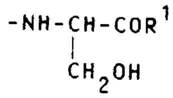

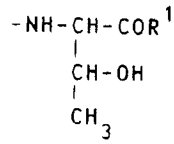

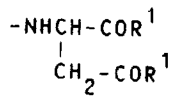

$-NH-CH_2-CH_2-COR^1$

$-N(CH_2COR^1)_2$

$-NH-(CH_2)_m-(C_6H_4)_n-SO_2R^1$

mit $R^1$ in der Bedeutung einer Hydroxygruppe oder eines

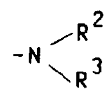

-Restes,

worin

$R^2$ und $R^3$ unabhängig voneinander ein Wasserstoffatom, eine gerade oder verzweigte, gesättigte oder ungesättigte, gegebenenfalls durch 1 bis 5 Hydroxy- oder $C_1$-$C_4$-Alkoxygruppen substituierte Alkylgruppe mit 1 bis 16 Kohlenstoffatomen oder eine Aryl- oder Aralkylgruppe, oder

$R^2$ und $R^3$ gemeinsam mit dem Stickstoff einen gegebenenfalls ein weiteres Stickstoff-, Sauerstoff-, Schwefelatom oder eine Carbonylgruppe enthaltenden gesättigten oder ungesättigten Fünf- oder Sechsring bedeuten,

steht.

Als Alkylsubstituenten $R^2$ und $R^3$ kommen gesättigte. ungesättigte, gerad- oder verzweigtkettige oder cyclische Kohlenwasserstoffe mit bis zu 16 C-Atomen, vorzugsweise gesättigte Kohlenwasserstoffe mit bis zu 7 Kohlenstoffatomen, in Betracht, die gegebenenfalls durch 1 bis 5 Hydroxy- oder niedere Alkoxygruppen substituiert sind.

Niedere Alkoxygruppen sollen jeweils 1 bis 4 Kohlenstoffatome enthalten und insbesondere Methoxy- und Ethoxygruppen umfassen.

Als gegebenenfalls substituierte Alkylgruppen seien beispielsweise die Methyl-, Ethyl-, Hydroxymethyl-, 1-Hydroxyethyl-, 2-Hydroxyethyl-, Z-Hydroxy-1-(hydroxymethyl)-ethyl-, 1-(Hydroxymethyl)-ethyl-, Propyl-, Isopropyl-, Propenyl-, Isopropenyl-, 2- und 3-Hydroxypropyl-, 2,3-Dihydroxypropyl-, Butyl-, Isobutenyl-, 2-, 3- und 4-Hydroxybutyl-, 2-, 3- und 4-Hydroxy-2-methylbutyl-, 2- und 3-Hydroxy-isobutyl-, 2,3,4-Trihydroxybutyl-, Cyclohexyl-, Pentyl-, Hexyl-, Bis- und Tris(hydroxymethyl)methyl-, 2,3-Dihydroxy-1-(hydroxymethyl)propyl-, 2,3,4,5,6-Pentahydroxyhexyl, 1,3,4-trihydroxybutyl-2- und 2-Methoxyethylgruppe genannt.

Bevorzugt sind unsubstituierte Alkylgruppen mit 1 bis 7 Kohlenstoffatomen, wie zum Beispiel die Methyl-, Ethyl-, n-Propyl-, Isopropyl-, n-Butyl-, Isobutyl-, Pentyl- und Hexylgruppe. Ferner sind bevorzugt mono- und poly-hydroxysubstituierte Alkylgruppen mit 2 bis 7 Kohlenstoffatomen und 1 bis 5, vorzugsweise 1 bis 4 Hydroxygruppen, wie zum Beispiel 2- und 3-Hydroxypropyl, 1 ,3-Dihydroxyisopropyl, 1- (Hydroxymethyl)-ethyl, Bis- und Tris(hydroxymethyl)methyl, 2,3-Dihydroxy-1-hydroxymethylpropyl, 2,3,4,5,6-Pentahydroxyhexyl und vorzugsweise 2-Hydroxy-ethyl, 2-Hydroxy-1-(hydroxymethyl)-ethyl, 2,3-Dihydroxypropyl und 2,3,4-Trihydroxybutyl.

Steht $R^2$ bzw. $R^3$ für eine Aryl- oder Aralkylgruppe, so ist die Phenyl- bzw. Benzylgruppe bevorzugt.

Der durch $R^2$ und $R^3$ unter Einschluß des Amid-Stickstoffs gebildete heterocyclische 5- oder 6-Ring kann gesättigt oder ungesättigt sein und gegebenenfalls ein Stickstoff-, Sauerstoff-, Schwefelatom oder eine Carbonylgruppe enthalten.

Als geeignete Heterocyclen seien beispielhaft genannt: der Pyrrolidinyl-, Piperidyl-, Pyrazolidinyl-, Piperidonyl-, Pyrrolinyl-, Pyrazolinyl-, Piperazinyl-, Morpholinyl-, Imidazolidinyl-, Oxazolidinyl-, Thiazolidinyl-Ring.

Der -CO-Y- und $-SO_2R^1$-Substituent steht bevorzugt in der o- oder p-Position des Benzolrings.

Die in den Verbindungen der allgemeinen Formel I vorhandenen aciden Wasserstoffatome können gegebenenfalls ganz oder teilweise durch Kationen anorganischer und/oder organischer Basen oder Aminosäuren ersetzt sein. Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der Fluorbenzolsulfonamide der allgemeinen Formel I erfolgt dadurch, daß man in an sich bekannter Weise Verbindungen der allgemeinen Formel II

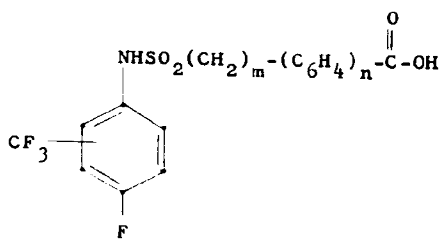

(II),

- gegebenenfalls in aktivierter Form -

mit Verbindungen der allgemeinen Formel III

H-Y′ (III),

worin

Y′ für den Rest einer gegebenenfalls geschützten Aminocarbon- oder Aminosulfonsäure oder für den Rest eines Aminocarbon- oder Aminosulfonsäureamids, worin gegebenenfalls vorhandene Hydroxygruppen gegebenenfalls geschützt sind,

umsetzt,

anschließend die Schutzgruppen entfernt, gewünschtenfalls die Säuregruppen mit organischen oder anorganischen Basen in die entsprechenden Salze überführt oder gegebenenfalls nach Aktivierung der in Y enthaltenen Säuregruppen, gewünschtenfalls mit einem Amin der allgemeinen Formel IV

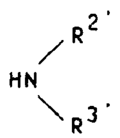

(IV),

worin

$R^{2'}$ und $R^{3'}$ die für $R^2$ und $R^3$ angegebene Bedeutung haben, wobei darin gegebenenfalls enthaltene Hydroxygruppen gegebenenfalls geschützt sind,

umsetzt und gegebenenfalls vorhandene Schutzgruppen entfernt.

Als Säureschutzgruppen kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Säuren können auch in Form ihrer Salze, vorzugsweise als Ammoniumsalz, eingesetzt werden.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Als Hydroxyschutzgruppen kommen alle diejenigen infrage, die sich leicht einführen und sich später unter Rückbildung der letztlich gewünschten freien Hydroxygruppe auch wieder leicht abspalten lassen. Bevorzugte Schutzgruppen sind Ethergruppen wie z.B. die Benzyl-, 4-Methoxybenzyl-, 4-Nitrobenzyl-, Di- und Triphenylmethyl, Trimethylsilyl-, Dimethyl-t-butylsilyl, Diphenyl-t-butylsilylgruppe.

Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle

von Polyolen können die Hydroxygruppen auch in Form von Ketalen mit z.B. Aceton, Acetaldehyd, Cyclohexanon oder Benzaldehyd geschützt sein.

Die Abspaltung der Hydroxy-Schutzgruppen erfolgt in an sich bekannter Weise, z.B. im Falle eines Benzylethers, durch reduktive Spaltung mit Lithium/Ammoniak oder durch hydrogenolytische Spaltung in Gegenwart von z.B. Palladium-Kohle, im Falle eines Esters z.B. durch alkalische Verseifung in wäßrig-alkoholischer Lösung bei Temperaturen von 0 bis 50 °C bzw. bei tert.-Butylestern mit Hilfe von Trifluoressigsäure, sowie im Falle einer Ether- oder Ketalspaltung durch Säurebehandlung mit Hilfe von z.B. Kationenaustauschern, Trifluoressigsäure oder Mineralsäuren (siehe z.B. "Protective Groups in Organic Synthesis", T.W. Greene, John Wiley and Sons 1981).

Als Beispiel für eine aktivierte Carboxylgruppe seien Anhydrid, p-Nitrophenylester und Säurechlorid genannt.

Die Umsetzung der Carbonsäuren der allgemeinen Formel II mit den Verbindungen der allgemeinen Formel III H-Y′ bzw. der so erhaltenen Fluorbenzolsulfonamide der allgemeinen Formel I, worin Y für den Rest einer Aminocarbon- oder Aminosulfonsäure steht, mit Aminen der allgemeinen Formel IV $HNR^{2'}R^{3'}$ erfolgt nach literaturbekannten Methoden, z.B. in Gegenwart von Reagenzien wie Carbodiimid, vorzugsweise Dicyclohexylcarbodiimid (DCC), (z.B. Am. Soc 81, 890) in aprotischen Lösungsmitteln wie z.B. Dimethylformamid, Dioxan, Dichlormethan oder deren Gemische bei Temperaturen von -10 °C bis 100 °C, vorzugsweise -10 °C bis Raumtemperatur, innerhalb von 1 bis 24, vorzugsweise 2 bis 12 Stunden.

Die Knüpfung der Amidbindungen kann auch durch Aminolyse von aktivierten Carboxylgruppen mit Verbindungen der allgemeinen Formel III bzw. IV erfolgen.

So erfolgt die Aminolyse von z.B. Estern in flüssiger Phase, z.B. in einem geeigneten höhersiedenden Lösungsmittel wie Dimethylformamid, Dimethylacetamid oder Dimethylsulfoxid. Die Reaktionstemperaturen liegen bei etwa 20 °C - 200 °C, wobei Temperaturen von 100 °C - 180 °C bevorzugt sind. Die Reaktionszeiten liegen zwischen 2 Stunden und 2 Tagen, wobei Reaktionszeiten zwischen 4 Stunden und 36 Stunden bevorzugt sind.

Darüber hinaus können alle dem Fachmann bekannten Methoden zur Umwandlung von Carboxylgruppen in Amidgruppen zur Synthese der erfindungsgemäßen Fluorbenzolsulfonamide der allgemeinen Formel I herangezogen werden, so z.B. die Methode nach Krejcarek und Tucker, Biochem. Biophys. Res. Commun. 77, 581 (1977) über gemischte Anhydride.

Als geeignete Amine der allgemeinen Formel IV seien beispielsweise genannt: Dimethylamin, Diethylamin, Di-n-propylamin, Diisopropylamin, Di-n-butylamin, Diisobutylamin, Di-sek.-butylamin, N-Methyl-n-propylamin, Dioctylamin, Dicyclohexylamin, N-Ethylcyclohexylamin, Diisopropenylamin, Benzylamin, Anilin, 4-Methoxyanilin, 4-Dimethylaminoanilin, 3,5-Dimethoxyanilin, Morpholin, Pyrrolidin, Piperidin, N-Methylpiperazin, N-Ethyl-piperazin, N-(2-Hydroxyethyl)-piperazin, N-(Hydroxymethyl)-piperazin, Piperazinoessigsäureisopropylamid, N-(Piperazinomethylcarbonyl)morpholin, N-(Piperazinomethylcarbonyl)-pyrrolidin, 2-(2-Hydroxymethyl)piperidin, 4-(2-Hydroxyethyl)-piperidin, 2-Hydroxymethylpiperidin, 4-Hydroxymethylpiperidin, 2-Hydroxymethyl-pyrrolidin, 3-Hydroxy-piperidin, 4-Hydroxypiperidin, 3-Hydroxy-pyrrolidin, 4-Piperidon, 3-Pyrrolin, Piperidin-3-carbonsäureamid, Piperidin-4-carbonsäureamid, Piperidin-3-carbonsäurediethylamid, Piperidin-4-carbonsäuredimethylamid, 2,6-Dimethylpiperidin, 2,6-Dimethylmorpholin, N-Acetyl-piperazin, H- (2-Hydroxy-propionyl)-piperazin, N-(3-Hydroxy-propionyl)-piperazin, N-(Methoxyacetyl)-piperazin, 4-(N-Acetyl,N-methylamino)-piperidin, Piperidin-4-carbonsäure-(3-oxapentamethylen)amid, Piperidin-3-carbonsäure-(3-oxapentamethylen)-amid, N-(N′ ,N′-Dimethyl-carbamoyl)piperazin, Pyrazolin, Pyrazolidin, Imidazolin, Oxazolidin, Thiazolidin, 2,3-Dihydroxypropylamin, N-Methyl-2,3-dihydroxypropylamin, 2-Hydroxy-1-(hydroxymethyl)-ethylamin, N,N-Bis-(2-hydroxyethyl)-amin, N-Methyl-2,3,4,5,6-pentahydroxyhexylamin, 6-Amino-2,2-dimethyl-1,3-dioxepin-5-ol, 2-Hydroxyethylamin, 2-Amino-1,3-propandiol, Diethanolamin, Ethanolamin.

Die Polyhydroxyalkylamine können vorteilhafterweise auch in geschützter Form zur Reaktion eingesetzt werden, z.B. als O-Acylderivate oder als Ketale. Dies gilt besonders dann, wenn diese Derivate bequemer und billiger herstellbar sind als die Polyhydroxylalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 31 50 917.

Die nachträgliche Entfernung der Schutzgruppen ist problemlos und kann z.B. durch Behandlung mit einem sauren Ionenaustauscher in wäßrig-ethanolischer Lösung erfolgen.

Die Synthese der Edukte der allgemeinen Formel II erfolgt in an sich bekannter Weise (Houben-Weyl, "Methoden der organischen Chemie" Band IX, S. 343, 398, 547 und 557, Georg Thieme Verlag, Stuttgart, 1955) durch Umsetzung von 4-Fluor-2-trifluormethyl-anilin bzw. 4-Fluor-3-trifluormethyl-anilin mit Halogensulfonylderivaten (z.B. Chlorsulfonylessigester oder Chlorsulfonylbenzoesäure) in Gegenwart von Säurefängern wie z.B. tertiäres Amin [z.B. Triethylamin, Pyridin, N,N-Dimethylaminopyridin, 1,5-Diazabicyclo-[4.3.0]-nonen-5(DBN), 1,5-Diazabicyclo-[5.4.0]-undecen-5-(DBU)], Alkali-, Erdalkalicarbonat- oder -hydrogencarbonat (z.B.

Natrium-, Magnesium-, Calcium-, Barium-, Kalium-carbonat und -hydrogencarbonat) in Lösungsmitteln wie z.B. Dioxan, Dichlorethan oder Dichlormethan bei Temperaturen zwischen -20 bis +50 °C, vorzugsweise -5 bis 20 °C.

Die gewünschtenfalls durchzuführende Verseifung von so erhaltenen Estern folgt literaturbekannten Methoden. Wie bereits oben erwähnt, können jedoch auch die Ester als Ausgangsmaterial für die folgenden Reaktionen dienen.

Nach Herstellung der gewünschten Verbindung der allgemeinen Formel I können im Molekül vorhandene acide Wasserstoffatome durch Kationen anorganischer und/oder organischer Basen substituiert werden.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium und Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin.

Zur Herstellung der neutralen Salze kann man beispielsweise den Säuren in wäßriger Lösung oder Suspension ein Äquivalent der gewünschten Basen zusetzen. Die erhaltene Lösung kann anschließend im Vakuum zur Trockne eingeengt oder gefriergetrocknet werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1, 2-Dimethoxyethan und andere) auszufällen und so leicht zu isolierende und gut zu reinigende Kristalisate zu erhalten.

Enthalten die sauren Verbindungen mehrere freie acide Gruppen, so ist es oft zweckmäßig, neutrale Mischsalze herzustellen, die sowohl anorganische als auch organische Kationen als Gegenionen enthalten.

Die Herstellung der erfindungsgemäßen diagnostischen Mittel erfolgt ebenfalls in an sich bekannter Weise, indem man die erfindungsgemäßen Verbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween $^{(R)}$, Myrj$^{(R)}$) und/oder Aromastoff (en) zur Geschmackskorrektur (zum Beispiel ätherischen Ölen) gemischt.

Die erfindungsgemäßen fluorhaltigen Verbindungen können vorteilhaft in der in-vivo NMR-Diagnostik, d.h. für die NMR-Bildgebung und in der NMR-Spektroskopie als Indikator verschiedener Parameter eingesetzt werden. So können unter anderem mit Hilfe der ortsaufgelösten Spektroskopie und damit gewebsspezifisch pH, $pO_2$, $pCO_2$, Temperatur, Redox-Vorgänge, Reaktionskinetik gemessen werden.

Weiterhin wurde festgestellt, daß sich die erfindungsgemäßen Verbindungen überraschenderweise durch eine sehr gute Verträglichkeit auszeichnen.

Die erfindungsgemäßen pharmazeutischen Mittel werden vorzugsweise in einer Konzentration von 1 µMol-1 Mol/l hergestellt. Sie werden in der Regel in Mengen von 0,005 - 20 mMol/kg Körpergewicht, vorzugsweise 0,05 - 5 mMol/kg Körpergewicht, dosiert. Sie sind zur enteralen und parenteralen Applikation bestimmt.

Die erfindungsgemäßen Mittel erfüllen die vielfältigen Voraussetzungen für die Eignung als Diagnostika für die NMR-Tomographie und -Spektroskopie. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen, das heißt NMR-Diagnostika müssen 100 - 1000fach besser wasserlöslich sein als die in der in-vitro NMR-Spektroskopie verwendeten Mittel.

Die gute Verträglichkeit der erfindungsgemäßen Verbindungen erlaubt die Prüfung und NMR-spektroskopische Vermessung des pH-Wertes im lebenden Organismus. Hierbei ermöglicht eine Gabe von 10 µmol/kg bis 10 mmol/kg Körpergewicht eine problemlose Bestimmung der Veränderung der chemischen Verschiebung des $^{19}F$-Signals im Verhältnis zum Referenzsignal (z.B. einer intramolekularen $CF_3$-Gruppe) und damit des pH-Wertes. Die applizierte Lösung verteilt sich rasch im Organismus und ist somit in der Lage, Bereiche unterschiedlichen pH-Wertes nachzuweisen. Durch eine entsprechende Dosierung kann darüber hinaus eine Veränderung des pH-Wertes und damit gegebenenfalls ein therapeutischer Effekt bewirkt werden.

Um kleine Änderungen des pH-Wertes aufzeigen zu können, sind die Verbindungen von Vorteil, deren

pK-Wert in der Nähe des biologischen bzw. pathologischen pH-Wertes des interessierenden Gewebes ist. In der Regel sind diejenigen Verbindungen von besonderem Interesse, deren pK-Wert zwischen 2 und 9, vorzugsweise zwischen 6 und 8, liegt. Verbindungen, die den pH-Wert des Magen-Darm-Traktes aufzeigen, haben vorteilhafterweise einen pK zwischen 2 und 8. Da die größte Genauigkeit der pH-Bestimmung im Bereich der größten Veränderung der chemischen Verschiebung pro Einheit, also beim pK-Wert der jeweiligen Verbindung, vorliegt, ist eine sehr gute Analyse biologischer Vorgänge möglich. So liegt der pH des Blutes bei etwa 7.2 - 7.4; pathologische Bereiche können einen veränderten pH-Wert haben, der z.B. bis auf 4.0 oder niedriger sinken kann.

Für die Darstellung der Nierenfunktion bzw. Analyse des Primär- und Sekundärharns sind Verbindungen mit einem pK-Wert zwischen 5 und 7 von Vorteil, da der pH-Wert des Urins in der Regel unter dem des Blutes liegt. Für die Bestimmung des intragastralen pH-Wertes sind die Verbindungen von Vorteil, die eine Veränderung der chemischen Verschiebung zwischen pH 2 und 6 am deutlichsten zeigen, da der pH-Wert des Magensaftes zwischen fast 1 und 7 stark schwanken kann.

Durch die Verwendung der sehr gut verträglichen neuartigen fluorierten Meßsonden ist es somit möglich geworden, in kleineren Volumina (z.B. 10 ccm) ortsaufgelöste Spektroskopie durchzuführen und physiologisch wichtige Parameter wie z.B. den pH-Wert präzise in kurzer Meßzeit ohne Störung bzw. Überlagerung durch andere Moleküle zu bestimmen.

Für ein in-vivo Imaging (NMR-Bildgebung) sind die genannten Verbindungen ebenfalls geeignet. Hierbei werden nicht nur die Informationen der veränderten chemischen Verschiebung bildlich dargestellt, sondern es werden die lokalen Konzentrationen der fluorierten Verbindungen durch die in der MRT üblichen Aufnahmesequenzen in einem Imaging wiedergegeben. Der Vorteil des $^{19}$F-Imaging gegenüber der $^{1}$H-Tomographie beruht darin, daß die Verteilung des Pharmakons direkt ohne Überlagerung durch störende Strukturen dargestellt werden kann.

So gelingt z.B. eine hervorragende Kontrastierung des renalen Ausscheidungssystems (Niere, Urether, Blase) nach intravenöser Gabe der erfindungsgemäßen Verbindungen, welche in einer Dosis von 5 µmol/kg bis 20 mmol/kg, vorzugsweise von 0,1 mmol/kg bis 5 mmol/kg, appliziert werden. Hierbei wurde überraschenderweise auch gefunden, daß die zusätzliche Injektion einer paramagnetischen Verbindung (z.B. GdDTPA/Dimeglumin) in einer Dosis von 1 µmol/kg bis 2 mmol/kg, vorzugsweise von 50 µmol/kg bis 500 µmol/kg, zu einer deutlichen Verbesserung der Bildqualität führt.

Gekoppelt an Makromoleküle, zum Beispiel monoklonale Antikörper, können die erfindungsgemäßen Verbindungen auch Verwendung als organ- und tumorspezifische Therapeutika und Diagnostika finden.

Ist in den erfindungsgemäßen Verbindungen das $^{18}$F-Isotop enthalten, dann sind diese als Diagnostika für die Positronenemissions-Tomographie (PET) geeignet.

Die erfindungsgemäßen Verbindungen der allgemeinen Formel I sind auch gegen alle bakteriellen Infektionen, die chemotherapeutisch mit Sulfonamiden zu behandeln sind, einzusetzen.

Die nachfolgenden Beispiele dienen zur näheren Erläuterung des Erfindungsgegenstandes.

**Beispiel 1**

2-{2-[N-(4-Fluor-2-trifluormethylphenyl)-sulfamoyl]-acetylamino}-essigsäure

a) **2-{2-[N-(4-Fluor-2-trifluormethylphenyl)-sulfamoyl]-acetylamino}-essigsäureethylester**

In 100 ml Dimethylformamid werden 1,561 g (5 mmol) 2-[N-(4-Fluor-2-trifluormethylphenyl)-sulfamoyl]-essigsäure, 700 mg (5 mmol) Glycinethylester Hydrochlorid und 766 mg (5 mmol) Hydroxybenztriazol, Hydrat gegeben und unter Rühren auf -12° abgekühlt. Dann gibt man 506 mg (5 mmol) Triethylamin dazu, läßt 10 Minuten rühren und versetzt mit 1,03 g (5 mmol) Dicyclohexylcarbodiimid. Man läßt auftauen und rührt über Nacht. Der Feststoff wird abgesaugt und man engt im Vakuum zur Trockene ein. Der Rückstand wird zwischen Wasser und Dichlormethan verteilt. Die organische Lösung wird über Natriumsulfat getrockent und im Vakuum zur Trockene eingeengt. Der Rückstand wird aus Ethanol kristallisiert.
Ausbeute: 1,932 g (77,7 % d. Theorie).
Schmelzpunkt: 128 - 30°C.

Analyse: $C_{13}H_{14}F_4N_2O_5S$ (386,32)

| | | | | | | | |
|---|---|---|---|---|---|---|---|
| C 40,42 | H 3,65 | F 19,67 | N 7,25 | O 20,71 | S 8,30 | Ber. |
| C 40,71 | H 3,61 | F 19,54 | N 7,33 | O - | S 8,21 | Gef. |

b) **2-{2-[N-(4-Fluor-2-trifluormethylphenyl)-sulfamoyl]-acetylamino}-essigsäure**

In 25 ml warmen Ethanol werden 1,30 g (3,37 mmol) des unter Beispiel 1a hergestellten Ethylesters gelöst und mit 5 ml (10 mmol) 2 N Natriumlauge versetzt. Man erwärmt unter Rühren bis kein Ausgangsmaterial mehr nachzuweisen ist, säuert mit Salzsäure auf einen pH-Wert von 3 an und engt im Vakuum zur Trockene ein. Die Substanz wird in Essigester aufgenommen und auch daraus kristallisiert.
Ausbeute: 960 mg (79,6 % d. Theorie)
Schmelzpunkt: 168 - 70°C.

Analyse: $C_{11}H_{10}F_4N_2O_5S$ (358,27)

| | | | | | | |
|---|---|---|---|---|---|---|
| C 36,88 | H 2,81 | F 21,21 | N 7,82 | O 22,33 | S 8,95 | Ber. |
| C 37,05 | H 2,96 | F 21,09 | N 7,76 | O - | S 8,84 | Gef. |

**Beispiel 2**

2-{2-[N-(4-Fluor-3-trifluormethylphenyl)-sulfamoyl]-acetylamino}-essigsäure

a) **2-{2-[N-(4-Fluor-3-trifluormethylphenyl)-sulfamoyl]-acetylamino}-essigsäureethylester**

In 100 ml Dimethylformamid werden unter Stickstoff 1,81 g (6 mmol) 2-[N-(4-Fluor-3-trifluormethylphenyl)-sulfamoyl]-essigsäure, 0,845 g (6 mmol) Glycinethylester, Hydrochlorid (98 %) und 0,919 g (6 mmol) Hydroxybenztriazol Hydrat gegeben und unter Rühren auf -10° gekühlt. Dann gibt man 0,607 g (6 mmol) Triethylamin dazu, läßt 10 Minuten rühren und versetzt mit 1,24 g (6 mmol) Dicyclohexylcarbodiimid. Man läßt noch eine Stunde bei der tiefen Temperatur rühren und dann ohne Kühlung über Nacht. Man saugt vom Feststoff ab, engt die Lösung im Vakuum zur Trockene ein und verteilt den Rückstand zwischen Natriumbicarbonatlösung und Essigester. Die Essigesterlösung wird mit Wasser gewaschen, über Natriumsulfat getrocknet und im Vakuum zur Trockene eingeengt. Der Rückstand wird aus Essigester/Hexan kristallisiert. Man erhält 1,72 g (74,1 % d. Theorie) der Titelverbindung.
Schmelzpunkt: 145 - 7°C.

Analyse: $C_{13}H_{14}F_4N_2O_5S$ (386,32)

| | | | | | | |
|---|---|---|---|---|---|---|
| C 40,42 | H 3,66 | F 19,67 | N 7,25 | O 20,71 | S 8,30 | Ber. |
| C 40,46 | H 3,78 | F 19,57 | N 7,34 | O - | S 8,37 | Gef. |

b) **2-{2-[N-(4-Fluor-3-trifluormethylphenyl)-sulfamoyl]-acetylamino}-essigsäure**

In 20 ml Dioxan werden 1,66 g (4,30 mmol) der unter Beispiel 2a hergestellten Verbindung gelöst. Man versetzt mit 5,4 ml (10,8 mmol) 2N Natronlauge, erwärmt auf dem Wasserbad bis kein Ausgangsmaterial mehr nachzuweisen ist und stellt dann mit Salzsäure einen pH-Wert von 3 ein. Die Lösung wird im Vakuum zur Trockene eingeengt, der Rückstand wird zwischen Essigester und Wasser verteilt. Die Essigesterlösung wird über Natriumsulfat getrocknet und im Vakuum zur Trockene eingeengt. Die Titelverbindung wird aus Essigester/Hexan kristallisiert.
Ausbeute: 1,271 g (82,50 % d. Theorie)
Schmelzpunkt: 146 - 48°C.

Analyse: $C_{11}H_{10}F_4N_2O_5S$ (358,26)

| | | | | | | |
|---|---|---|---|---|---|---|
| C 36,88 | H 2,81 | F 21,21 | N 7,82 | O 22,33 | S 8,95 | Ber. |
| C 36,82 | H 3,01 | F 21,01 | N 7,78 | O - | S 8,83 | Gef. |

**Beispiel 3**

2-{2-[N-(4-Fluor-2-trifluormethylphenyl)-sulfamoyl]-acetylamino}-2-hydroxymethylessigsäure

a) **2-{2-[N-(4-Fluor-2-trifluormethylphenyl)-sulfamoyl]-acetylamino}-2-hydroxymethylessigsäuremethylester**

Unter den Bedingungen des Beispiels 2a werden 3,29 g (10,93 mmol) 2-[N-(4-Fluor-2-trifluormethylphenyl)-sulfamoyl]-essigsäure, 1,70 g (10,93 mmol) L-Serin-methylester Hydrochlorid, 1,67 g (10,93 mmol) Hydroxybenztriazol Hydrat sowie 1,11 g (10,93 mmol) Triethylamin und 2,26 g (10,93 mmol) Dicyclohexylcarbodiimid umgesetzt und in analoger Weise aufgearbeitet. Das Rohprodukt wird an Kieselgel chromatographiert. Die Titelverbindung wird mit Ethanol eluiert. Sie kristallisiert beim Einengen.
Ausbeute: 3,44 g (78,2 % d. Theorie)
Schmelzpunkt: 143 - 45°C.

Analyse: $C_{13}H_{14}F_4N_2O_6S$ (402,32)

| | | | | | | |
|---|---|---|---|---|---|---|
| C 38,81 | H 3,51 | F 18,89 | N 6,96 | O 23,86 | S 7,97 | Ber. |
| C 39,00 | H 3,45 | F 18,80 | N 6,92 | O - | S 8,07 | Gef. |

b) **2-{2-[N-(4-Fluor-2-trifluormethylphenyl)-sulfamoyl]-acetylamino}-2-hydroxymethyl-essigsäure**

Unter den Bedingungen des Beispiels 2b werden 1,91 g (4,75 mmol) des unter Beispiel 3a hergestellten Esters mit 5,94 ml (11,88 mmol) 2N Natronlauge in 20 ml Dioxan verseift. Man erhält nach analoger Aufarbeitung einen Feststoff, der aus Essigester/Hexan umkristallisiert wird. Die Ausbeute an der Titelverbindung beträgt 1,49 g (80,7 % d. Theorie).
Schmelzpunkt: 145 - 47°C.

Analyse: $C_{12}H_{12}F_4N_2O_6S$ (388,29)

| | | | | | | |
|---|---|---|---|---|---|---|
| C 37,12 | H 3,12 | F 19,57 | N 7,21 | O 24,72 | S 8,26 | Ber. |
| C 37,02 | H 3,16 | F 19,75 | N 7,19 | O - | S 8,42 | Gef. |

**Beispiel 4**

2-{2-[N-(4-Fluor-3-trifluormethylphenyl)-sulfamoyl]-acetylamino}-2-hydroxymethylessigsäure

a) **2-{2-[N-(4-Fluor-3-trifluormethylphenyl)-sulfamoyl]-acetylamino}-2-hydroxymethyl-essigsäuremethylester**

Unter den Bedingungen des Beispiels 2a werden 3,29 g (10,93 mmol) 2-[N-(4-Fluor-3-trifluormethylphenyl)-sulfamoyl]-essigsäure, 1,70 g (10,93 mmol) L-Serin-methylester Hydrochlorid, 1,67 g (10,93 mmol) Hydroxybenztriazol Hydrat sowie 1,11 g (10,93 mmol) Triethylamin und 2,26 g (10,93 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,17 g (72,1 % d. Theorie)

Analyse: $C_{13}H_{14}F_4N_2O_6S$ (402,32)

| | | | | | | |
|---|---|---|---|---|---|---|
| C 38,81 | H 3,51 | F 18,89 | N 6,96 | O 23,86 | S 7,97 | Ber. |
| C 39,09 | H 3,55 | F 18,77 | N 7,18 | O - | S 7,99 | Gef. |

b) **2-{2-[N-(4-Fluor-3-trifluormethylphenyl)-sulfamoyl]-acetylamino}-2-hydroxymethylessigsäure**

In 30 ml Dioxan werden 2,30 g (5,72 mmol) des unter Beispiel 4a hergestellten Esters gelöst und mit 6 ml (12 mmol) 2N Bariumhydroxidlösung versetzt. Man läßt 24 Stunden bei Raumtemperatur rühren (kein Ausgangsmaterial mehr nachweisbar), stellt mit Salzsäure einen pH-Wert von 5 ein und engt im Vakuum zur Trockene ein. Der Rückstand wird zwischen Essigester und angesäuertem Wasser verteilt. Die Essigesterlösung wird über Natriumsulfat getrocknet und im Vakuum zur Trockene eingeengt. Man erhält die Titelverbindung als dünnschichtchromatographisch einheitliches Material. Die Ausbeute beträgt 1,613 g (72,6 % der Theorie).

| Analyse: | $C_{12}H_{12}F_4N_2O_6S$ (388,29) | | | | | |
|---|---|---|---|---|---|---|
| C 37,12 | H 3,12 | F 19,57 | N 7,21 | O 24,72 | S 8,62 | Ber. |
| C 37,03 | H 3,18 | F 19,44 | N 7,16 | O - | S 8,20 | Gef. |

**Beispiel 5**

2-{4-[N-(4-Fluor-3-trifluormethylphenyl)-sulfamoyl]-benzoylamino}-2-hydroxymethylessigsäure

a) **2-{4-[N-(4-Fluor-3-trifluormethylphenyl)-sulfamoyl]-benzoylamino}-2-hydroxymethylessigsäuremethylester**

Unter den Bedingungen des Beispiels 2a werden 3,60 g (9,91 mmol) 4-[N-(4-Fluor-3-trifluormethylphenyl)-sulfamoyl]-benzoesäure, 1,54 g (9,91 mmol) L-Serin-methylester Hydrochlorid, 1,52 g (9,91 mmol) Hydroxybenztriazol Hydrat sowie 1,00 g (9,91 mmol) Triethylamin und 2,04 g (9,91 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,88 g (84,3 % d. Theorie).

| Analyse: | $C_{18}H_{16}F_4N_2O_6S$ (464,39) | | | | | |
|---|---|---|---|---|---|---|
| C 46,56 | H 3,47 | F 16,36 | N 6,03 | O 20,67 | S 6,90 | Ber. |
| C 46,46 | H 3,69 | F 16,31 | N 5,90 | O - | S 6,79 | Gef. |

b) **2-{4-[N-(4-Fluor-3-trifluormethylphenyl)-sulfamoyl]-benzoylamino}-2-hydroxymethylessigsäure**

Unter den Bedingungen des Beispiels 4b werden 3,82 g (8,23 mmol) des unter 5a hergestellten Esters in 40 ml Dioxan mit 8,5 ml (17 mmol) 2N Bariumhydroxidlösung verseift. Man erhält nach analoger Aufarbeitung die Titelverbindung als dünnschichtchromatographisch einheitliches Material.
Die Ausbeute beträgt 2,74 g (73,9 % d.Theorie).

| Analyse: | $C_{11}H_{14}F_4N_2O_6S$ (450,36) | | | | | |
|---|---|---|---|---|---|---|
| C 45,34 | H 3,13 | F 16,87 | N 6,22 | O 21,32 | S 7,12 | Ber. |
| C 45,39 | H 3,18 | F 16,70 | N 6,28 | O - | S 7,05 | Gef. |

**Beispiel 6**

2-{2-[N-(4-Fluor-2-trifluormethylphenyl)-sulfamoyl]-acetylamino}-2-methyl-essigsäure

a) **2-{2-[N-(4-Fluor-2-trifluormethylphenyl)-sulfamoyl]-acetylamino}-2-methyl-essigsäureethylester**

Unter den Bedingungen des Beispiels 2a werden 3,012 g (10 mmol) 2-[N-(4-Fluor-2-trifluormethylphenyl)-sulfamoyl]-essigsäure, 1,536 g (10 mmol) L-Alaninethylester Hydrochlorid, 1,531 g (10 mmol) Hydroxybenztriazol Hydrat sowie 1,012 g (10 mmol) Triethylamin umgesetzt und in analoger Weise aufgearbeitet. Das Rohprodukt wird an Kieselgel chromatographiert. Die Titelverbindung wird mit Essigester eluiert.
Ausbeute: 2,99 g (74,6 % d. Theorie)

Analyse: $C_{14}H_{10}F_4N_2O_5S$ (400,35)

| | | | | | | |
|---|---|---|---|---|---|---|
| C 42,00 | H 4,03 | F 18,98 | N 7,00 | O 19,98 | S 8,01 | Ber. |
| C 41,83 | H 4,11 | F 18,90 | N 7,07 | O - | S 7,89 | Gef. |

b) **2-{2-[N-(4-Fluor-2-trifluormethylphenyl)-sulfamoyl]-acetylamino}-2-methyl-essigsäure**

Unter den Bedingungen des Beispiels 2b werden 3,203 g (8 mmol) des unter 6a hergestellten Esters mit 10 ml (20 mmol) 2N Natronlauge in 40 ml Dioxan verseift. Man arbeitet in analoger Weise auf und erhält 2,418 g (81,2 % der Theorie) der Titelverbindung.

Analyse: $C_{12}H_{12}F_4N_2O_5S$ (372,29)

| | | | | | | |
|---|---|---|---|---|---|---|
| C 38,72 | H 3,25 | F 20,41 | N 7,52 | O 21,49 | S 8,61 | Ber. |
| C 38,81 | H 3,20 | F 20,33 | N 7,47 | O - | S 8,52 | Gef. |

**Beispiel 7**

2-{2-[N-(4-Fluor-2-trifluormethylphenyl)-sulfamoyl]-acetylamino}-2-(1-hydroxyethyl)-essigsäure

a) **2-{2-[N-(4-Fluor-2-trifluormethylphenyl)-sulfamoyl]-acetylamino}-2-(1-hydroxyethyl)-essigsäureethylester**

Unter den Bedingungen des Beispiels 2a werden 3,012 g (10 mmol) 2-[N-(4-Fluor-2-trifluormethylphenyl)-sulfamoyl]-essigsäure, 1,736 g (10 mmol) L-Threoninethylester Hydrochlorid, 1,531 g (10 mmol) Hydroxybenztriazol Hydrat sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Ethanol eluiert.
Ausbeute: 3,41 g (79,2 % d. Theorie)

Analyse: $C_{15}H_{18}F_4N_2O_6S$ (430,37)

| | | | | | | |
|---|---|---|---|---|---|---|
| C 41,86 | H 4,22 | F 17,66 | N 6,51 | O 22,31 | S 7,45 | Ber. |
| C 41,95 | H 4,30 | F 17,55 | N 6,46 | O - | S 7,42 | Gef. |

b) **2-{2-[N-(4-Fluor-2-trifluormethylphenyl)-sulfamoyl]-acetylamino}-2-(1-hydroxyethyl)-essigsäure**

Unter den Bedingungen des Beispiels 4b werden 1,72 g (4 mmol) des unter 7a hergestellten Esters mit 4,5 ml (9 mmol) 2N Bariumhydroxidlösung in 20 ml Dioxan verseift. Man arbeitet in analoger Weise zu 2b auf und erhält 1,20 g (74,6 % d. Theorie) der Titelverbindung.

Analyse: $C_{13}H_{14}F_4N_2O_6S$ (402,32)

| | | | | | | |
|---|---|---|---|---|---|---|
| C 38,81 | H 3,51 | F 18,89 | N 6,96 | O 23,86 | S 7,97 | Ber. |
| C 38,73 | H 3,45 | F 18,96 | N 7,03 | O - | S 7,90 | Gef. |

**Beispiel 8**

3-{2-[N-(4-Fluor-2-trifluormethylphenyl)-sulfamoyl]-acetylamino}-3-carboxy-propionsäure

a) **3-{2-[N-(4-Fluor-2-trifluormethylphenyl)-sulfamoyl]-acetylamino}-3-methoxycarbonyl-propionsäuremethylester**

Unter den Bedingungen des Beispiels 2a werden 3,012 g (10 mmol) 2-[N-(4-Fluor-2-trifluormethylphenyl)-sulfamoyl]-essigsäure, 1,836 g (10 mmol) L-Asparaginsäuredimethylester Hydrochlorid, 1,531 g (10 mmol) Hydroxybenztriazol Hydrat sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,573 g (80,4 % der Theorie)

Analyse: $C_{15}H_{16}F_4N_2O_7S$ (444,36)

| | | | | | | |
|---|---|---|---|---|---|---|
| C 40,55 | H 3,63 | F 17,10 | N 6,30 | O 25,20 | S 7,22 | Ber. |
| C 40,59 | H 3,72 | F 17,02 | N 6,37 | O - | S 7,11 | Gef. |

b) **3-{2-[N-(4-Fluor-2-trifluormethylphenyl)-sulfamoyl]-acetylamino}-3-carboxy-propionsäure**

Unter den Bedingungen des Beispiels 2b werden 2,222 g (5 mmol) des unter Beispiel 8a hergestellten Esters mit 8 ml (16 mmol) 2N Natronlauge in 20 ml Dioxan verseift. Man arbeitet in analoger Weise zu Beispiel 2b auf und erhält 1,755 g (84,3 % der Theorie) der Titelverbindung.

Analyse: $C_{13}H_{12}F_4N_2O_7S$ (416,30)

| | | | | | | |
|---|---|---|---|---|---|---|
| C 37,51 | H 2,91 | F 18,25 | N 6,73 | O 26,90 | S 7,70 | Ber. |
| C 37,38 | H 2,98 | F 18,21 | N 6,68 | O - | S 7,61 | Gef. |

**Beispiel 9**

3-{2-[N-(4-Fluor-2-trifluormethylphenyl)-sulfamoyl]-acetylamino}-propionsäure

a) **3-{2-[N-(4-Fluor-2-trifluormethylphenyl)-sulfamoyl]-acetylamino}-propionsäure-ethylester**

Unter den Bedingungen des Beispiels 2a werden 3,012 g (10 mmol) 2-[N-(4-Fluor-2-trifluormethylphenyl)-sulfamoyl]-essigsäure, 1,536 g (10 mmol) β-Alaninethylester Hydrochlorid, 1,531 g (10 mmol) Hydroxybenztriazol Hydrat sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatogaphie an Kieselgel gereinigt. Die Titelverbindung wird mit Essigester eluiert.
Ausbeute: 3,259 g (81,4 % der Theorie).

Analyse: $C_{14}H_{16}F_4N_2O_5S$ (400,35)

| | | | | | | |
|---|---|---|---|---|---|---|
| C 42,00 | H 4,03 | F 18,98 | N 7,00 | O 19,98 | S 8,01 | Ber. |
| C 42,08 | H 4,11 | F 18,89 | N 7,05 | O - | S 7,88 | Gef. |

b) **3-{2-[N-(4-Fluor-2-trifluormethylphenyl)-sulfamoyl]-acetylamino}-propionsäure**

Unter den Bedingungen des Beispiels 2b werden 2,40 g (6 mmol) des unter Beispiel 9a hergestellten Esters mit 6,5 ml (13 mmol) 2N Natronlauge in 25 ml Dioxan verseift. Es wird in analoger Weise zu Beispiel 2b aufgearbeitet, und man erhält 1,725 g (77,2 % der Theorie) der Titelverbindung.

Analyse: $C_{12}H_{12}F_4N_2O_5S$ (372,29)

| | | | | | | | |
|---|---|---|---|---|---|---|---|
| C 38,72 | H 3,25 | F 20,41 | N 7,52 | O 21,49 | S 8,61 | Ber. |
| C 38,81 | H 3,27 | F 20,32 | N 7,61 | O - | S 8,54 | Gef. |

**Beispiel 10**

2-{2-[N-(4-Fluor-2-trifluormethylphenyl)-sulfamoyl]-acetylimino}-diessigsäure

a) **2-{2-[N-(4-Fluor-2-trifluormethylphenyl)-sulfamoyl]-acetylimino}-diessigsäurediethylester**

Unter den Bedingungen des Beispiels 2a werden 3,012 g (10 mmol) 2-[N-(4-Fluor-2-trifluormethylphenyl)-sulfamoyl]-essigsäure, 2,257 g (10 mmol) Iminodiessigsäurediethylester Hydrochlorid, 1,531 g (10 mmol) Hydroxybenztriazol Hydrat sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Ethanol eluiert.
Ausbeute: 4,035 g (85,4 % der Theorie)

Analyse: $C_{17}H_{20}F_4N_2O_7S$ (472,41)

| | | | | | | |
|---|---|---|---|---|---|---|
| C 43,22 | H 4,27 | F 16,09 | N 5,93 | O 23,71 | S 6,79 | Ber. |
| C 43,20 | H 4,35 | F 16,00 | N 5,98 | O - | S 6,85 | Gef. |

b) **2-{2-[N-(4-Fluor-2-trifluormethylphenyl)-sulfamoyl]-acetylimino}-diessigsäure**

Unter den Bedingungen des Beispiels 2b werden 3,307 g (7 mmol) des unter 10a hergestellten Esters mit 12 ml (24 mmol) 2N Natronlauge in 40 ml Dioxan verseift. Man arbeitet in analoger Weise auf und erhält 2,436 g (83,6 % der Theorie) der Titelverbindung.

Analyse: $C_{13}H_{12}F_4N_2O_7S$ (416,30)

| | | | | | | |
|---|---|---|---|---|---|---|
| C 37,51 | H 2,91 | F 18,25 | N 6,73 | O 26,90 | S 7,70 | Ber. |
| C 37,38 | H 3,00 | F 18,18 | N 6,80 | O - | S 7,59 | Gef. |

pKa (37 °C): 6,68 ppm/pH: 4,94 $LD_{50}$ [mmol/kg] , Maus: $\leqq$ 7,5

**Beispiel 11**

2-{4-(N-(4-Fluor-3-trifluormethylphenyl)-sulfamoyl]-benzoylamino}-essigsäure

a) **2-{4-[N-(4-Fluor-3-trifluormethylphenyl)-sulfamoyl]-benzoylamino}-essigsäure-ethylester**

Unter den Bedingungen des Beispiels 2a werden 3,633 g (10 mmol) 4-[N-(4-Fluor-3-trifluormethylphenyl)-sulfamoyl]-benzoesäure, 1,396 g (10 mmol) Glycinethylester Hydrochlorid, 1,531 g (10 mmol) Hydroxybenztriazol Hydrat sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,69 g (82,3 % d. Theorie)

Analyse: $C_{18}H_{16}F_4N_2O_5S$ (448,39)

| | | | | | | |
|---|---|---|---|---|---|---|
| C 48,22 | H 3,60 | F 16,95 | N 6,25 | O 17,84 | S 7,15 | Ber. |
| C 48,15 | H 3,71 | F 16,87 | N 6,20 | O - | S 7,20 | Gef. |

b) **2-{4-[N-(4-Fluor-3-trifluormethylphenyl)-sulfamoyl]-benzoylamino}-essigsäure**

Unter den Bedingungen des Beispiels 2b werden 3,139 g (7 mmol) des unter Beispiel 11a hergestellten Esters mit 8 ml (16 mmol) 2N Natronlauge in 30 ml Dioxan verseift. Man arbeitet wie unter Beispiel 2b beschrieben auf und erhält 2,369 g (80,5 % d. Theorie) der Titelverbindung.

Analyse: $C_{16}H_{12}F_4N_2O_5S$ (420,34)

| | | | | | | |
|---|---|---|---|---|---|---|
| C 45,72 | H 2,88 | F 18,08 | N 6,66 | O 19,03 | S 7,63 | Ber. |
| C 45,81 | H 2,97 | F 18,01 | N 6,58 | O - | S 7,55 | Gef. |

**Beispiel 12**

2-{4-[N-(4-Fluor-2-trifluormethylphenyl)-sulfamoyl]-benzoylamino}-essigsäure

a) **2-{4-[N-(4-Fluor-2-trifluormethylphenyl)-sulfamoyl]-benzoylamino}-essigsäure-ethylester**

Unter den Bedingungen des Beispiels 2a werden 3,633 g (10 mmol) 4-[N-(4-Fluor-2-trifluormethylphenyl)-sulfamoyl]-benzoesäure, 1,396 g (10 mmol) Glycinethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,553 g (79,2 % der Theorie)

Analyse: $C_{18}H_{16}F_4N_2O_5S$ (448,39)

| | | | | | | |
|---|---|---|---|---|---|---|
| C 48,22 | H 3,60 | F 16,95 | N 6,25 | O 17,84 | S 7,15 | Ber. |
| C 48,13 | H 3,67 | F 17,00 | N 6,31 | O - | S 7,08 | Gef. |

b) **2-{4-[N-(4-Fluor-2-trifluormethylphenyl)-sulfamoyl]-benzoylamino}-essigsäure**

Unter den Bedingungen des Beispiels 2b werden 3,139 g (7 mmol) des unter Beispiel 12a hergestellten Esters mit 8 ml (16 mmol) 2N Natronlauge in 30 ml Dioxan verseift. Man arbeitet in analoger Weise auf und erhält 2,313 g (78,6 % der Theorie) der Titelverbindung.

Analyse: $C_{16}H_{12}F_4N_2O_5S$ (420,34)

| | | | | | | |
|---|---|---|---|---|---|---|
| C 45,72 | H 2,88 | F 18,08 | N 6,66 | O 19,03 | S 7,63 | Ber. |
| C 45,65 | H 2,97 | F 18,15 | N 6,60 | O - | S 7,55 | Gef. |

**Beispiel 13**

2-{2-[N-(4-Fluor-2-trifluormethylphenyl)-sulfamoyl]-benzoylamino}-essigsäure

a) **2-{2-[N-(4-Fluor-2-trifluormethylphenyl)-sulfamoyl]-benzoylamino}-essigsäure-ethylester**

Unter den Bedingungen des Beispiels 2a werden 3,633 g (10 mmol) 2-[N-(4-Fluor-2-trifluormethylphenyl)-sulfamoyl]-benzoesäure, 1,396 g (10 mmol) Glycinethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,52 g (78,5 % der Theorie)

| | | | | | | | |
|---|---|---|---|---|---|---|---|
| Analyse: | $C_{18}H_{16}F_4N_2O_5S$ (448,39) | | | | | | |
| C 48,22 | H 3,60 | F 16,95 | N 6,25 | O 17,84 | S 7,15 | Ber. |
| C 48,29 | H 3,54 | F 16,98 | N 6,21 | O - | S 7,23 | Gef. |

b) **2-{2-[N-(4-Fluor-2-trifluormethylphenyl)-sulfamoyl]-benzoylamino}-essigsäure**

Unter den Bedingungen des Beispiels 2b werden 4,036 g (9 mmol) des unter Beispiel 13a hergestellten Esters mit 10 ml (20 mmol) 2N Natronlauge in 40 ml Dioxan verseift. Man arbeitet in analoger Weise auf und erhält 2,905 g (76,8 % der Theorie) der Titelverbindung.

| | | | | | | |
|---|---|---|---|---|---|---|
| Analyse: | $C_{16}H_{12}F_4N_2O_5S$ (420,34) | | | | | |
| C 45,72 | H 2,88 | F 16,08 | N 6,66 | O 19,03 | S 7,63 | Ber. |
| C 45,78 | H 2,93 | F 16,17 | N 6,69 | O - | S 7,52 | Gef. |

**Beispiel 14**

2-{2-[N-(4-Fluor-3-trifluormethylphenyl)-sulfamoyl]-benzoylamino}-essigsäure

a) **2-{2-[N-(4-Fluor-3-trifluormethylphenyl)-sulfamoyl]-benzoylamino}-essigsäure-ethylester**

Unter den Bedingungen des Beispiels 2a werden 3,633 g (10 mmol) 2-[N-(4-Fluor-3-trifluormethylphenyl)-sulfamoyl]-benzoesäure, 1,396 g (10 mmol) Glycinethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,448 g (76,9 % der Theorie)

| | | | | | | |
|---|---|---|---|---|---|---|
| Analyse: | $C_{18}H_{16}F_4N_2O_5S$ (448,39) | | | | | |
| C 48,22 | H 3,60 | F 16,95 | N 6,25 | O 17,84 | S 7,15 | Ber. |
| C 48,33 | H 3,72 | F 17,04 | N 6,33 | O - | S 7,04 | Gef. |

b) **2-{2-[N-(4-Fluor-3-trifluormethylphenyl)-sulfamoyl]-benzoylamino}-essigsäure**

Unter den Bedingungen des Beispiels 2b werden 2,69 g (6 mmol) des unter Beispiel 14a hergestellten Esters mit 6,5 ml (13 mmol) 2N Natronlauge in 25 ml Dioxan verseift. Man arbeitet in analoger Weise auf und erhält 1,977 g (78,4 % der Theorie) der Titelverbindung.

| | | | | | | |
|---|---|---|---|---|---|---|
| Analyse: | $C_{16}H_{12}F_4N_2O_5S$ (420,34) | | | | | |
| C 45,72 | H 2,88 | F 18,08 | N 6,66 | O 19,03 | S 7,63 | Ber. |
| C 45,80 | H 2,95 | F 18,01 | N 6,59 | O - | S 7,60 | Gef. |

**Beispiel 15**

2-{4-[N-(4-Fluor-2-trifluormethylphenyl)-sulfamoyl]-benzoylamino}-2-methyl-essigsäure

a) **2-{4-[N-(4-Fluor-2-trifluormethylphenyl)-sulfamoyl]-benzoylamino]-2-methyl-essigsäureethylester**

Unter den Bedingungen des Beispiels 2a werden 3,633 g (10 mmol) 4-[N-(4-Fluor-2-trifluormethylphenyl)-sulfamoyl]-benzoesäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,672 g (79,4 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,29 | H 3,98 | F 16,45 | N 6,11 | O - | S 6,88 | Gef. |

b) **2-{4-[N-(4-Fluor-2-trifluormethylphenyl)-sulfamoyl]-benzoylamino}2-methyl-essigsäure**

Unter den Bedingungen des Beispiels 2b werden 3,237 g (7 mmol) des unter Beispiel 15a hergestellten Esters mit 7,5 ml (15 mmol) 2N Natronlauge in 30 ml Dioxan verseift. Man arbeitet in analoger Weise auf und erhält 2,399 g (78,9 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 46,93 | H 3,32 | F 17,44 | N 6,51 | O - | S 7,30 | Gef. |

**Beispiel 16**

2-{4-[N-(4-Fluor-3-trifluormethylphenyl)-sulfamoyl]-benzoylamino}-2-methylessigsäure

a) **2-{4-[N-(4-Fluor-3-trifluormethylphenyl)-sulfamoyl]-benzoylamino}-2-methyl-essigsäureethylester**

Unter den Bedingungen des Beispiels 2a werden 3,633 g (10 mmol) 2-[N-(4-Fluor-3-trifluormethylphenyl)-sulfamoyl]-benzoesäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,593 g (77,7 % der Theorie)

Analyse: $C_{19}H_{16}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,30 | H 3,96 | F 16.40 | N 6,10 | O - | S 6,88 | Gef. |

b) **2-{4-[N-(4-Fluor-3-trifluormethylphenyl)-sulfamoyl]-benzoylamino}-2-methyl-essigsäure**

Unter den Bedingungen des Beispiels 2b werden 2,312 g (5 mmol) des unter Beispiel 16a hergestellten Esters mit 3 ml (6 mmol) 2N Natronlauge in 20 ml Dioxan verseift. Man arbeitet in analoger Weise auf und erhält 1,677 g (77,2 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 47,11 | H 3,30 | F 17,60 | N 6,36 | O - | S 7,29 | Gef. |

**Beispiel 17**

2-{2-[N-(4-Fluor-3-trifluormethylphenyl)-sulfamoyl]-benzoylamino}-2-methyl-essigsäure

a) **2-{2-[N-(4-Fluor-3-trifluormethylphenyl)-sulfamoyl]-benzoylamino}-2-methyl-essigsäureethylester**

Unter den Bedingungen des Beispiels 2a werden 3,633 g (10 mmol) 2-[N-(4-Fluor-3-trifluormethylphenyl)-sulfamoyl]-benzoesäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,625 g (78,4 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,42 | H 3,99 | F 16,38 | N 6,11 | O - | S 6,86 | Gef. |

b) **2-{2-[N-(4-Fluor-3-trifluormethylphenyl)-sulfamoyl]-benzoylamino}-2-methyl-essigsäure**

Unter den Bedingungen des Beispiels 2b werden 2,775 g (6 mmol) des unter Beispiel 17a hergestellten Esters mit 6,5 ml (13 mmol) 2N Natronlauge in 25 ml Dioxan verseift. Man arbeitet in analoger Weise auf und erhält 2,004 g (76,9 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 47,08 | H 3,32 | F 17,40 | N 6,51 | O - | S 7,28 | Gef. |

**Beispiel 18**

2-{2-[N-(4-Fluor-2-trifluormethylphenyl)-sulfamoyl]-benzoylamino}-2-methyl-essigsäure

a) **2-{2-[N-(4-Fluor-2-trifluormethylphenyl)-sulfamoyl]-benzoylamino}-2-methyl-essigsäureethylester**

Unter den Bedingungen des Beispiels 2a werden 3,633 g (10 mmol) 2-[N-(4-Fluor-2-trifluormethylphenyl)-sulfamoyl]-benzoesäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,796 g (82,1 % der Theorie)

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,50 | H 3,55 | F 16,30 | N 6,10 | O - | S 6,85 | Gef. |

b) **2-{2-[N-(4-Fluor-2-trifluormethylphenyl)-sulfamoyl]-benzoylamino}-2-methyl-essigsäure**

Unter den Bedingungen des Beispiels 2b werden 3,70 g (8 mmol) des unter Beispiel 18a hergestellten Esters mit 8,5 ml (17 mmol) 2N Natronlauge in 35 ml Dioxan verseift. Man arbeitet in analoger Weise auf und erhält 2,804 g (80,7 % der Theorie) der Titelverbindung.

Analyse: $C_{17}H_{14}F_4N_2O_6S$ (450,37)

| | | | | | | |
|---|---|---|---|---|---|---|
| C 45,34 | H 3,13 | F 16,87 | N 6,22 | O 21,32 | S 7,12 | Ber. |
| C 45,30 | H 3,18 | F 16,81 | N 6,27 | O - | S 7,09 | Gef. |

**Beispiel 19**

2-{4-[N-(4-Fluor-2-trifluormethylphenyl)-sulfamoyl]-benzoylamino}-2-hydroxymethyl-essigsäure

a) **2-{4-[N-(4-Fluor-2-trifluormethylphenyl)-sulfamoyl]-benzoylamino}-2-hydroxymethyl-essigsäuremethylester**

Unter den Bedingungen des Beispiels 2a werden 3,633 g (10 mmol) 4-[N-(4-Fluor-2-trifluormethylphenyl)-sulfamoyl]-benzoesäure, 1,556 g (10 mmol) L-Serinmethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,478 g (74,9 % der Theorie)

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,62 | H 3,54 | F 16,30 | N 6,09 | O - | 6,87 | Gef. |

b) **2-{4-[N-(4-Fluor-2-trifluormethylphenyl)-sulfamoyl]-benzoylamino}-2-hydroxymethyl-essigsäure**

Unter den Bedingungen des Beispiels 4b werden 2,786 g (6 mmol) des unter Beispiel 19a hergestellten Esters mit 6,5 ml (13 mmol) 2N Bariumhydroxydlösung in 25 ml Dioxan verseift. Man arbeitet in analoger Weise auf und erhält 2,119 g (78,4 % der Theorie) der Titelverbindung.

Analyse: $C_{17}H_{14}F_4N_2O_6S$ (450,37)

| | | | | | | |
|---|---|---|---|---|---|---|
| C 45,34 | H 3,13 | F 16,87 | N 6,22 | O 21,32 | S 7,12 | Ber. |
| C 45,26 | H 3,18 | F 16,81 | N 6,29 | O - | S 7,07 | Gef. |

**Beispiel 20**

2-{2-[N-(4-Fluor-3-trifluormethylphenyl)-sulfamoyl]-benzoylamino}-2-hydroxymethyl-essigsäure

a) **2-{2-[N-(4-Fluor-3-trifluormethylphenyl)-sulfamoyl]-benzoylamino}-2-hydroxymethyl-essigsäuremethylester**

Unter den Bedingungen des Beispiels 2a werden 3,633 g (10 mmol) 2-[N-(4-Fluor-3-trifluormethylphenyl)-sulfamoyl]-benzoesäure, 1,556 g (10 mmol) L-Serinmethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,646 g (78,5 % der Theorie)

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,60 | H 3,52 | F 16,38 | N 6,11 | O - | S 6,92 | Gef. |

b) **2-{2-[N-(4-Fluor-3-trifluormethylphenyl)-sulfamoyl]-benzoylamino}-2-hydroxymethyl-essigsäure**

Unter den Bedingungen des Beispiels 4b werden 2,090 g (4,5 mmol) des unter Beispiel 20a hergestellten Esters mit 5 ml (10 mmol) 2N Bariumhydroxidlösung in 20 ml Dioxan verseift. Man arbeitet in analoger Weise auf und erhält 1,552 g (76,6 % der Theorie) der Titelverbindung.

Analyse: $C_{17}H_{14}F_4N_2O_6S$ (450,37)

| | | | | | | |
|---|---|---|---|---|---|---|
| C 45,34 | H 3,13 | F 16,87 | N 6,22 | O 21,32 | S 7,12 | Ber. |
| C 45,27 | H 3,19 | F 16,80 | N 6,29 | O - | S 7,03 | Gef. |

**Beispiel 21**

2-{2-[N-(4-Fluor-2-trifluormethylphenyl)-sulfamoyl]-benzoylamino}-2-hydroxymethylessigsäure

a) **2-{2-[N-(4-Fluor-2-trifluormethylphenyl)-sulfamoyl]-benzoylamino}-2-hydroxymethyl-essigsäuremethylester**

Unter den Bedingungen des Beispiels 2a werden 3,633 g (10 mmol) 2-[N-(4-Fluor-2-trifluormethylphenyl)-sulfamoyl]-benzoesäure, 1,556 g (10 mmol) L-Serinmethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,673 g (79,1 % der Theorie)

Analyse: $C_{14}H_{16}F_4N_2O_5S$ (400,35)

| | | | | | | |
|---|---|---|---|---|---|---|
| C 42,00 | H 4,03 | F 18,98 | N 7,00 | O 19,98 | S 8,01 | Ber. |
| C 41,88 | H 4,09 | F 19,91 | N 7,02 | O - | S 8,05 | Gef. |

b) **2-{2-[N-(4-Fluor-2-trifluormethylphenyl)-sulfamoyl]-benzoylamino}-2-hydroxymethyl-essigsäure**

Unter den Bedingungen des Beispiels 4b werden 3,251 g (7 mmol) des unter Beispiel 21a hergestellten Esters mit 8 ml (16 mmol) 2N Bariumhydroxidlösung in 35 ml Dioxan verseift. Man arbeitet in analoger Weise auf und erhält 2,465 g (78,2 % der Theorie) der Titelverbindung.

Analyse: $C_{12}H_{12}F_4N_2O_5S$ (372,29)

| | | | | | | |
|---|---|---|---|---|---|---|
| C 38,72 | H 3,25 | F 20,41 | N 7,52 | O 21,49 | S 8,61 | Ber. |
| C 38,68 | H 3,29 | F 20,35 | N 7,44 | O - | S 8,53 | Gef. |

**Beispiel 22**

2-{2-[N-(4-Fluor-3-trifluormethylphenyl)-sulfamoyl]-benzoylamino}-2-methyl-essigsäure

a) **2-{2-[N-(4-Fluor-3-trifluormethylphenyl)-sulfamoyl]-benzoylamino}-2-methyl-essigsäureethylester**

Unter den Bedingungen des Beispiels 2a werden 3,012 g (10 mmol) 2-[N-(4-Fluor-3-trifluormethylphenyl)-sulfamoyl]-essigsäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.

Ausbeute: 3,055 g (76,3 % der Theorie)

Analyse: $C_{17}H_{20}F_4N_2O_7S$ (472,41)

| | | | | | | |
|---|---|---|---|---|---|---|
| C 43,22 | H 4,27 | F 16,09 | N 5,93 | O 23,71 | S 6,79 | Ber. |
| C 43,27 | H 4,34 | F 16,03 | N 5,88 | O - | S 6,72 | Gef. |

b) **2-{2-[N-(4-Fluor-3-trifluormethylphenyl)-sulfamoyl]-benzoylamino}-2-methyl-essigsäure**

Unter den Bedingungen des Beispiels 2b werden 3,003 g (7,5 mmol) des unter Beispiel 22a hergestellten Esters mit 8 ml (16 mmol) 2N Natronlauge in 35 ml Dioxan verseift. Man arbeitet in analoger Weise auf und erhält 2,195 g (78,6 % der Theorie) der Titelverbindung.

Analyse: $C_{13}H_{12}F_4N_2O_7S$ (416,30)

| | | | | | | |
|---|---|---|---|---|---|---|
| C 37,51 | H 2,91 | F 18,25 | N 6,73 | O 26,90 | S 7,70 | Ber. |
| C 37,60 | H 2,99 | F 18,20 | N 6,62 | O - | S 7,62 | Gef. |

**Beispiel 23**

2-{2-[N-(4-Fluor-3-trifluormethylphenyl)-sulfamoyl]-acetylamino}-2-(1-hydroxyethyl)-essigsäure

a) **2-{2-[N-(4-Fluor-3-trifluormethylphenyl)-sulfamoyl]-acetylamino}-2-(1-hydroxyethyl)-essigsäureethylester**

Unter den Bedingungen des Beispiels 2a werden 3,012 g (10 mmol) 2-{N-(4-Fluor-3-trifluormethylphenyl)-sulfamoyl]-essigsäure, 1,736 g (10 mmol) L-Threoninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,391 g (78,8 % der Theorie)

Analyse: $C_{15}H_{18}F_4N_2O_6S$ (430,37)

| | | | | | | |
|---|---|---|---|---|---|---|
| C 41,86 | H 4,22 | F 17,66 | N 6,51 | O 22,31 | S 7,45 | Ber. |
| C 41,80 | H 4,28 | F 17,62 | N 6,57 | O - | S 7,49 | Gef. |

b) **2-{2-[N-(4-Fluor-3-trifluormethylphenyl)-sulfamoyl]-acetylamino}-2-(1-hydroxyethyl)-essigsäure**

Unter den Bedingungen des Beispiels 4b werden 3,142g(7,3 mmol) des unter Beispiel 23a hergestellten Esters mit 8 ml (16 mmol) 2N Bariumhydroxidlösung in 30 ml Dioxan verseift. Es wird in analoger Weise zu Beispiel 2b aufgearbeitet und man erhält 2,285 g (77,8 % der Theorie) der Titelverbindung.

Analyse: $C_{13}H_{14}F_4N_2O_6S$ (402,32)

| | | | | | | |
|---|---|---|---|---|---|---|
| C 38,81 | H 3,51 | F 18,89 | N 6,96 | O 23,86 | S 7,97 | Ber. |
| C 38,84 | H 3,59 | F 18,81 | N 6,89 | O - | S 7,99 | Gef. |

**Beispiel 24**

3-{2-[N-(4-Fluor-3-trifluormethylphenyl)-sulfamoyl]-acetylamino}-3-carboxypropionsäure

a) **3-{2-[N-(4-Fluor-3-trifluormethylphenyl)-sulfamoyl]-acetylamino}-3-methoxycarbonyl-propionsäuremethylester**

Unter den Bedingungen des Beispiels 2a werden 3,012 g (10 mmol) 2-[N-(4-Fluor-3-trifluormethylphenyl)-sulfamoyl]-essigsäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,475 g (78,2 % der Theorie)

Analyse: $C_{15}H_{16}F_4N_2O_7S$ (444,36)

| | | | | | | |
|---|---|---|---|---|---|---|
| C 40,55 | H 3,63 | F 17,10 | N 6,30 | O 25,20 | S 7,22 | Ber. |
| C 40,48 | H 3,65 | F 17,00 | N 6,24 | O - | S 7,15 | Gef. |

b) **3-{2-[N-(4-Fluor-3-trifluormethylphenyl)-sulfamoyl]-acetylamino}-3-carboxy-propionsäure**

Unter den Bedingungen des Beispiels 2b werden 2,888 g (6,5 mmol) des unter Beispiel 24a hergestellten Esters mit 11 ml (22 mmol) 2N Natronlauge in 40 ml Dioxan verseift. Es wird in analoger Weise zu Beispiel 2b aufgearbeitet und man erhält 2,219 g (82,0 % der Theorie) der Titelverbindung.

Analyse: $C_{13}H_{12}F_4N_2O_7S$ (416,30)

| | | | | | | |
|---|---|---|---|---|---|---|
| C 37,51 | H 2,91 | F 18,25 | N 6,73 | O 26,90 | S 7,70 | Ber. |
| C 37,58 | H 2,99 | F 18,20 | N 6,70 | O - | S 7,64 | Gef. |

**Beispiel 25**

3-{2-[N-(4-Fluor-3-trifluormethylphenyl)-sulfamoyl]-acetylamino}-propionsäure

a) **3-{2-[N-(4-Fluor-3-trifluormethylphenyl)-sulfamoyl]-acetylamino}-propionsäure-ethylester**

Unter den Bedingungen des Beispiels 2a werden 3,012 g (10 mmol) 2-[N-(4-Fluor-3-trifluormethylphenyl)-sulfamoyl]-essigsäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,27 g (81,9 % der Theorie)

Analyse: $C_{14}H_{16}F_4N_2O_5S$ (400,35)

| | | | | | | |
|---|---|---|---|---|---|---|
| C 42,00 | H 4,03 | F 18,98 | N 7,00 | O 19,98 | S 8,01 | Ber. |
| C 41,95 | H 4,10 | F 18,89 | N 7,10 | O - | S 8,06 | Gef. |

b) **3-{2-[N-(4-Fluor-3-trifluormethylphenyl)-sulfamoyl]-acetylamino}-propionsäure**

Unter den Bedingungen des Beispiels 2b werden 2,522 g (6,3 mmol) des unter Beispiel 25a hergestellten Esters mit 7 ml (14 mmol) 2N Natronlauge in 25 ml Dioxan verseift. Es wird in analoger Weise zu Beispiel 2b

aufgearbeitet und man erhält 1,872 g (79,8 % der Theorie) der Titelverbindung.

Analyse: $C_{12}H_{12}F_4N_2O_5S$ (372,29)

| | | | | | | |
|---|---|---|---|---|---|---|
| C 38,72 | H 3,25 | F 20,41 | N 7,52 | O 21,49 | S 8,61 | Ber. |
| C 38,77 | H 3,30 | F 20,44 | N 7,48 | O - | S 8,60 | Gef. |

**Beispiel 26**

2-{2-[N-(4-Fluor-3-trifluormethylphenyl)-sulfamoyl]-acetylimino}-diessigsäure

a) **2-{2-[N-(4-Fluor-3-trifluormethylphenyl)-sulfamoyl]-benzoylimino}-diessigsäure-diethylester**

Unter den Bedingungen des Beispiels 2a werden 3,012 g (10 mmol) 2-[N-(4-Fluor-3-trifluormethylphenyl)-sulfamoyl]-essigsäure, 2,257 g (10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,812 g (80,7 % der Theorie)

Analyse: $C_{20}H_{20}F_4N_2O_6S$ (492,45)

| | | | | | | |
|---|---|---|---|---|---|---|
| C 48,78 | H 4,09 | F 15,43 | N 5,69 | O 19,49 | S 6,51 | Ber. |
| C 48,85 | H 4,17 | F 15,38 | N 5,66 | O - | S 6,45 | Gef. |

b) **2-{2-[N-(4-Fluor-3-trifluormethylphenyl)-sulfamoyl]-acetylimino}-diessigsäure**

Unter den Bedingungen des Beispiels 2b werden 3,307 g (7 mmol) des unter Beispiel 26a hergestellten Esters mit 12 ml (24 mmol) 2N Natronlauge in 35 ml Dioxan verseift. Es wird in analoger Weise zu Beispiel 2b aufgearbeitet und man erhält 2,404 g (82,5 % der Theorie) der Titelverbindung.

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,60 | H 3,52 | F 16,30 | N 6,00 | O - | S 6,83 | Gef. |

**Beispiel 27**

2-{4-[N-(4-Fluor-3-trifluormethylphenyl)-sulfamoyl]-benzoylamino}-2-(1-hydroxyethyl)-essigsäure

a) **2-{4-[N-(4-Fluor-3-trifluormethylphenyl)-sulfamoyl]-benzoylamino}-2-(1-hydroxyethyl)-essigsäure-ethylester**

Unter den Bedingungen des Beispiels 2a werden 3,633 g (10 mmol) 4-[N-(4-Fluor-3-trifluormethylphenyl)-sulfamoyl]-benzoesäure, 1,736 g (10 mmol) L-Threoninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,954 g (80,3 % der Theorie)

| Analyse: | $C_{20}H_{20}F_4N_2O_6S$ (492,45) | | | | | |
|---|---|---|---|---|---|---|
| C 48,78 | H 4,09 | F 15,43 | N 5,69 | O 19,49 | S 6,51 | Ber. |
| C 48,86 | H 4,15 | F 15,38 | N 5,77 | O - | S 6,55 | Gef. |

b) **2-{4-[N-(4-Fluor-3-trifluormethylphenyl)-sulfamoyl]-benzoylamino}-2-(1-hydroxyethyl)-essigsäure**

Unter den Bedingungen des Beispiels 4b werden 2,61 g (5,3 mmol) des unter Beispiel 27a hergestellten Esters mit 6 ml (12 mmol) 2N Bariumhydroxidlösung in 25 ml Dioxan verseift. Man arbeitet in analoger Weise auf und erhält 1,935 g (78,6 % der Theorie) der Titelverbindung.

| Analyse: | $C_{18}H_{16}F_4N_2O_6S$ (464,39) | | | | | |
|---|---|---|---|---|---|---|
| C 46,54 | H 3,47 | F 16,36 | N 6,03 | O 20,67 | S 6,90 | Ber. |
| C 46,66 | H 3,54 | F 16,45 | N 6,08 | O - | S 6,79 | Gef. |

**Beispiel 28**

2-{4-[N-(4-Fluor-2-trifluormethylphenyl)-sulfamoyl]-benzoylamino}-2-(1-hydroxyethyl)-essigsäure

a) **2-{4-[N-(4-Fluor-2-trifluormethylphenyl)-sulfamoyl]-benzoylamino}-2-(1-hydroxyethyl)-essigsäure-ethylester**

Unter den Bedingungen des Beispiels 2a werden 3,633 g (10 mmol) 4-[N-(4-Fluor-2-trifluormethylphenyl)-sulfamoyl]-benzoesäure, 1736 g (10 mmol) L-Threoninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,856 g (78,3 % der Theorie)

| Analyse: | $C_{20}H_{20}F_4N_2O_6S$ (492,45) | | | | | |
|---|---|---|---|---|---|---|
| C 48,78 | H 4,09 | F 15,43 | N 5,69 | O 19,49 | S 6,51 | Ber. |
| C 48,71 | H 4,15 | F 15,36 | N 5,62 | O - | S 6,43 | Gef. |

b) **2-{4-[N-(4-Fluor-2-trifluormethylphenyl)-sulfamoyl]-benzoylamino}-2-(1-hydroxyethyl)-essigsäure**

Unter den Bedingungen des Beispiels 4b werden 3,94 g (8 mmol) des unter Beispiel 28a hergestellten Esters mit 8,5 ml (17mmol) 2N Bariumhydroxidlösung in 40 ml Dioxan verseift. Man arbeitet in analoger Weise auf und erhält 2,905 g (78,2 % der Theorie) der Titelverbindung.

| Analyse: | $C_{18}H_{16}F_4N_2O_6S$ (464,39) | | | | | |
|---|---|---|---|---|---|---|
| C 46,56 | H 3,47 | F 16,36 | N 6,03 | O 20,67 | S 6,90 | Ber. |
| C 46,48 | H 3,53 | F 16,31 | N 6,07 | O - | S 6,85 | Gef. |

**Beispiel 29**

2- 2-[N-(4-Fluor-3-trifluormethylphenyl)-sulfamoyl]-benzoylamino -2-(1-hydroxyethyl)-essigsäure

a) **2- 2-[N-(4-Fluor-3-trifluormethylphenyl)-sulfamoyl]-benzoylamino -2-(1-hydroxyethyl)-essigsäure-ethylester**

Unter den Bedingungen des Beispiels 2a werden 3,663 g (10 mmol) 2-[N-(4-Fluor-3-trifluormethylphenyl)-sulfamoyl]-benzoesäure, 1,736,g (10 mmol) L-Threoninethylester Hydrochlorid, 1,531 g (10 mmol)Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,969 g (80,6 % der Theorie)

Analyse: $C_{20}H_{20}F_4N_2O_6S$ (492,45)

| | | | | | | |
|---|---|---|---|---|---|---|
| C 48,78 | H 4,09 | F 15,43 | N 5,69 | O 19,49 | S 6,51 | Ber. |
| C 48,76 | H 4,14 | F 15,40 | N 5,73 | O - | S 6,47 | Gef. |

b) **2- 2-[N-(4-Fluor-2-trifluormethylphenyl)-sulfamoyl]-benzoylamino -2-(1-hydroxyethyl)-essigsäure**

Unter den Bedingungen des Beispiels 4b werden 3,447 g (7 mmol) des unter Beispiel 29a hergestellten Esters mit 7,5 ml (15 mmol) 2N Bariumhyydroxidlösung in 30 ml Dioxan verseift. Man arbeitet in analoger Weise auf und erhält 2,581 g (79,4 % der Theorie) der Titelverbindung.

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,50 | H 3,49 | F 16,31 | N 6,08 | O - | S 6,84 | Gef. |

**Beispiel 30**

2-{2-[N-(4-Fluor-2-trifluormethylphenyl)-sulfamoyl]-benzoylamino}-2-(1-hydroxyethyl)-essigsäure

a) **2-{2-[N-(4-Fluor-2-trifluormethylphenyl)-sulfamoyl]-benzoylamino}-2-(1-hydroxyethyl)-essigsäure-ethylester**

Unter den Bedingungen des Beispiels 2a werden 3,633 g (10 mmol) 2-[N-(4-Fluor-2-trifluormethylphenyl)-sulfamoyl]-benzoesäure, 1,736 g (10 mmol) L-Threoninethylester, Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,881 g (78,8 % der Theorie)

Analyse: $C_{20}H_{20}F_4N_2O_6S$ (492,45)

| | | | | | | |
|---|---|---|---|---|---|---|
| C 48,78 | H 4,09 | F 15,43 | N 5,69 | O 19,49 | S 6,51 | Ber. |
| C 48,76 | H 4,14 | F 15,40 | N 5,73 | O - | S 6,47 | Gef. |

b) **2-{2-[N-(4-Fluor-2-trifluormethylphenyl)-sulfamoyl]-benzoylamino}-2-(1-hydroxyethyl)-essigsäure**

Unter den Bedingungen des Beispiels 4b werden 2,955 g (6 mmol) des unter Beispiel 30a hergestellten

Esters mit 6,5 ml (13 mmol) 2N Bariumhydroxidlösung in 30 ml Dioxan verseift. Man arbeitet in analoger Weise auf und erhält 2,143 g (76,9 % der Theorie) der Titelverbindung.

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,67 | S 6,90 | Ber. |
| C 46,50 | H 3,49 | F 16,31 | N 6,08 | O - | S 6,84 | Gef. |

**Beispiel 31**

3-{4-[N-(4-Fluor-3-trifluormethylphenyl)-sulfamoyl]-benzoylamino}-3-carboxy-propionsäure

a) **3-{4-[N-(4-Fluor-3-trifluormethylphenyl)-sulfamoyl]-benzoylamino}-3-methoxycarbonyl-propionsäuremethylester**

Unter den Bedingungen des Beispiels 2a werden 3,633 g (10 mmol) 4-[N-(4-Fluor-3-trifluormethylphenyl)-sulfamoyl]-benzoesäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,138 g (81,7 % der Theorie)

Analyse: $C_{20}H_{18}F_4N_2O_7S$ (506,43)

| | | | | | | |
|---|---|---|---|---|---|---|
| C 47,43 | H 3,58 | F 15,01 | N 5,53 | O 22,11 | S 6,33 | Ber. |
| C 47,37 | H 3,63 | F 15,04 | N 5,48 | O - | S 6,29 | Gef. |

b) **3- 4-[N-(4-Fluor-3-trifluormethylphenyl)-sulfamoyl]-benzoylamino -3-carboxy-propinsäure**

Unter den Bedingungen des Beispiels 2b werden 3,039 g (6 mmol) des unter Beispiel 31a hergestellten Esters mit 7 ml (14 mmol) 2N Natronlauge in 40 ml Dioxan verseift. Man arbeitet in analoger Weise auf und erhält 2,365 g (82,4 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,22 | H 3,00 | F 15,83 | N 5,79 | O - | S 6,63 | Gef. |

**Beispiel 32**

3-{4-[N-(4-Fluor-2-trifluormethylphenyl)-sulfamoyl]-benzoylamino}-3-carboxy-propionsäure

a) **3-{4-[N-(4-Fluor-2-trifluormethylphenyl)-sulfamoyl]-benzoylamino}-3-methoxycarbonyl-propionsäuremethylester**

Unter den Bedingungen des Beispiels 2a werden 3,633 g (10 mmol) 4-[N-(4-Fluor-2-trifluormethylphenyl)-sulfamoyl]-benzoesäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,046 g (79,9 % der Theorie)

Analyse: $C_{20}H_{18}F_4N_2O_7S$ (506,43)

| | | | | | | |
|---|---|---|---|---|---|---|
| C 47,43 | H 3,58 | F 15,01 | N 5,53 | O 22,11 | S 6,33 | Ber. |
| C 47,46 | H 3,69 | F 14,96 | N 5,48 | O - | S 6,27 | Gef. |

b) **3-{4-[N-(4-Fluor-2-trifluormethylphenyl)-sulfamoyl]-benzoylamino}-3-carboxy-propionsäure**

Unter den Bedingungen des Beispiels 2b werden 3,039 g (6 mmol) des unter Beispiel 32a hergestellten Esters mit 7 ml (14 mmol) 2N Natronlauge in 30 ml Dioxan verseift. Man arbeitet in analoger Weise auf und erhält 2,354 g (82,0 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,14 | H 2,99 | F 15,79 | N 5,90 | O - | S 6,59 | Gef. |

**Beispiel 33**

3-{2-[N-(4-Fluor-3-trifluormethylphenyl)-sulfamoyl]-benzoylamino}-propionsäure

a) **3-{2-[N-(4-Fluor-3-trifluormethylphenyl)-sulfamoyl]-benzoylamino}-propionsäure-ethylester**

Unter den Bedingungen des Beispiels 2a werden 3,633 g (10 mmol) 2-[N-(4-Fluor-3-trifluormethylphenyl)-sulfamoyl]-benzoesäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,806 g (82,3 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,40 | H 3,98 | F 16,40 | N 6,03 | O - | S 6,88 | Gef. |

b) **3-{2-[N-(4-Fluor-3-trifluormethylphenyl)-sulfamoyl]-benzoylamino}-propionsäure**

Unter den Bedingungen des Beispiels 2b werden 3,329 g (7,2 mmol) des unter Beispiel 33a hergestellten Esters mit 8 ml (16 mmol) 2N Natronlauge in 40 ml Dioxan verseift. Man arbeitet in analoger Weise auf und erhält 2,527 g (80,8 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 46,95 | H 3,31 | F 17,43 | N 6,50 | O - | S 7,33 | Gef. |

**Beispiel 34**

3-{2-[N-(4-Fluor-2-trifluormethylphenyl)-sulfamoyl]-benzoylamino}-propionsäure

a) **3-{2-[N-(4-Fluor-2-trifluormethylphenyl)-sulfamoyl]-benzoylamino}-propionsäure-ethylester**

Unter den Bedingungen des Beispiels 2a werden 3,633 g (10 mmol) 2-[N-(4-Fluor-2-trifluormethylphenyl)-sulfamoyl]-benzoesäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,733 g (81,6 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,31 | H 3,97 | F 16,39 | N 6,10 | O - | S 6,97 | Gef. |

b) **3-{2-[N-(4-Fluor-2-trifluormethylphenyl)-sulfamoyl]-benzoylamino}-propionsäure**

Unter den Bedingungen des Beispiels 2b werden 2,960 g (6,4 mmol) des unter Beispiel 34a hergestellten Esters mit 7 ml (14 mmol) 2N Natronlauge in 30 ml Dioxan verseift. Man arbeitet in analoger Weise auf und erhält 2,213 g (79,6 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 47,10 | H 3,31 | F 17,42 | N 6,52 | O - | S 7,28 | Gef. |

**Beispiel 35**

3-{2-[N-(4-Fluor-3-trifluormethylphenyl)-sulfamoyl]-benzoylamino}-3-carboxy-propionsäure

a) **3-{2-[N-(4-Fluor-3-trifluormethylphenyl)-sulfamoyl]-benzoylamino}-3-methoxycarbonyl-propionsäuremethylester**

Unter den Bedingungen des Beispiels 2a werden 3,633 g (10 mmol) 2-[N-(4-Fluor-3-trifluormethylphenyl)-sulfamoyl]-benzoesäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,107 g (81,1 % der Theorie)

Analyse: $C_{20}H_{18}F_4N_2O_7S$ (506,42)

| | | | | | | |
|---|---|---|---|---|---|---|
| C 47,43 | H 3,58 | F 15,01 | N 5,53 | O 22,11 | S 6,33 | Ber. |
| C 47,51 | H 3,66 | F 15,00 | N 5,48 | O - | S 6,27 | Gef. |

b) **3-{2-[N-(4-Fluor-3-trifluormethylphenyl)-sulfamoyl]-benzoylamino}-3-carboxy-propionsäure**

Unter den Bedingungen des Beispiels 2b werden 2,887 g (5,7 mmol) des unter Beispiel 35a hergestellten Esters mit 7 ml (14 mmol) 2N Natronlauge in 35 ml Dioxan verseift. Man arbeitet in analoger Weise auf und

erhält 2,203 g (80,8 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,14 | H 2,89 | F 15,82 | N 5,90 | O - | S 6,58 | Gef. |

**Beispiel 36**

3-{2-[N-(4-Fluor-2-trifluormethylphenyl)-sulfamoyl]-benzoylamino}-3-carboxy-propionsäure

a) **3-{2-[N-(4-Fluor-2-trifluormethylphenyl)-sulfamoyl]-benzoylamino}-3-methoxycarbonyl-propionsäuremethylester**

Unter den Bedingungen des Beispiels 2a werden 3,633 g (10 mmol) 2-[N-(4-Fluor-2-trifluormethylphenyl)-sulfamoyl]-benzoesäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,046 g (79,9 % der Theorie)

Analyse: $C_{20}H_{18}F_4N_2O_7S$ (506,43)

| | | | | | | |
|---|---|---|---|---|---|---|
| C 47,43 | H 3,58 | F 15,01 | N 5,53 | O 22,11 | S 6,33 | Ber. |
| C 47,38 | H 3,64 | F 14,94 | N 5,50 | O - | S 6,26 | Gef. |

b) **3-{2-[N-(4-Fluor-2-trifluormethylphenyl)-sulfamoyl]-benzoylamino}-3-carboxy-propionsäure**

Unter den Bedingungen des Beispiels 2b werden 3,039 g (6 mmol) des unter Beispiel 36a hergestellten Esters mit 6,5 ml (13 mmol) 2N Natronlauge in 30 ml Dioxan verseift. Man arbeitet in analoger Weise auf und erhält 2,265 g 78,9 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,31 | H 3,08 | F 15,74 | N 5,91 | O - | S 6,58 | Gef. |

**Beispiel 37**

3-{4-[N-(4-Fluor-3-trifluormethylphenyl)-sulfamoyl]-benzoylamino}-propionsäure

a) **3-{4-[N-(4-Fluor-3-trifluormethylphenyl)-sulfamoyl]-benzoylamino}-propionsäure-ethylester**

Unter den Bedingungen des Beispiels 2a werden 3,633 g (10 mmol) 2-[N-(4-Fluor-3-trifluormethylphenyl)-sulfamoyl]-benzoesäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,635 g (78,6 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,48 | H 3,98 | F 16,37 | N 6,12 | O - | S 6,85 | Gef. |

b) **3-{4-[N-(4-Fluor-3-trifluormethylphenyl)-sulfamoyl]-benzoylamino}-propionsäure**

Unter den Bedingungen des Beispiels 2b werden 2,405 g (5,2 mmol) des unter Beispiel 37a hergestellten Esters mit 6 ml (12 mmol) 2N Natronlauge in 25 ml Dioxan verseift. Man arbeitet in analoger Weise auf und erhält 1,843 g (81,6 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 47,08 | H 3,31 | F 17,49 | N 6,40 | O - | S 7,31 | Gef. |

**Beispiel 38**

3-{4-[N-(4-Fluor-2-trifluormethylphenyl)-sulfamoyl]-benzoylamino}-propionsäure

a) **3-{4-[N-(4-Fluor-2-trifluormethylphenyl)-sulfamoyl]-benzoylamino}-propionsäure-ethylester**

Unter den Bedingungen des Beispiels 2a werden 3,663 g (10 mmol) 4-[N-(4-Fluor-2-trifluormethylphenyl)-sulfamoyl]-benzoesäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 3,57 g (77,2 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,27 | H 3,96 | F 16,36 | N 6,11 | O - | S 6,84 | Gef. |

b) **3-{4-[N-(4-Fluor-2-trifluormethylphenyl)-sulfamoyl]-benzoylamino}-propionsäure**

Unter den Bedingungen des Beispiels 2b werden 2,821 g (6,1 mmol) des unter Beispiel 38a hergestellten Esters mit 7 ml (14 mmol) 2N Natronlauge in 30 ml Dioxan verseift. Man arbeitet in analoger Weise auf und erhält 2,08 g (78,5 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 46,90 | H 3,33 | F 17,40 | N 6,51 | O - | S 7,31 | Gef. |

**Beispiel 39**

2-{2-[N-(4-Fluor-2-trifluormethylphenyl)-sulfamoyl]-benzoylimino}-diessigsäure

a) **2-{2-[N-(4-Fluor-2-trifluormethylphenyl)-sulfamoyl]-benzoylimino}-diessigsäure-diethylester**

Unter den Bedingungen des Beispiels 2a werden 3,633 g (10 mmol) 2-[N-(4-Fluor-2-trifluormethylphenyl)-sulfamoyl]-benzoesäure, 2,257 g ( 10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,420 g (82,7 % der Theorie)

Analyse: $C_{22}H_{22}F_4N_2O_7S$ (534,48)

| | | | | | | |
|---|---|---|---|---|---|---|
| C 49,44 | H 4,15 | F 14,22 | N 5,24 | O 20,95 | S 6,00 | Ber. |
| C 49,40 | H 4,20 | F 14,13 | N 5,15 | O - | S 5,91 | Gef. |

b) **2-{2-[N-(4-Fluor-2-trifluormethylphenyl)-sulfamoyl]-benzoylimino}-diessigsäure**

Unter den Bedingungen des Beispiels 2b werden 3,207 g (6 mmol) des unter Beispiel 39a hergestellten Esters mit 7 ml (14 mmol) 2N Natronlauge in 30 ml Dioxan verseift. Man arbeitet in analoger Weise auf und erhält 2,279 g (79,4 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,28 | H 3,03 | F 15,82 | N 5,80 | O - | S 6,59 | Gef. |

**Beispiel 40**

2-{4-[N-(4-Fluor-3-trifluormethylphenyl)-sulfamoyl]-benzoylimino}-diessigsäure

a) **2-{4-[N-(4-Fluor-3-trifluormethylphenyl)-sulfamoyl]-benzoylimino}-diessigsäure-diethylester**

Unter den Bedingungen des Beispiels 2a werden 3,633 g (10 mmol) 4-[N-(4-Fluor-3-trifluormethylphenyl)-sulfamoyl]-benzoesäure, 2,257 g (10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,238 g (79,3 % der Theorie)

Analyse: $C_{22}H_{22}F_4N_2O_7S$ (534,48)

| | | | | | | |
|---|---|---|---|---|---|---|
| C 49,44 | H 4,15 | F 14,22 | N 5,24 | O 20,95 | S 6,00 | Ber. |
| C 49,50 | H 4,21 | F 14,12 | N 5,26 | O - | S 5,89 | Gef. |

b) **2-{4-[N-(4-Fluor-3-trifluormethylphenyl)-sulfamoyl]-benzoylimino}-diessigsäure**

Unter den Bedingungen des Beispiels 2b werden 3,10 g (5,8 mmol) des unter Beispiel 40a hergestellten Esters mit 7 ml (14 mmol) 2N Natronlauge in 30 ml Dioxan verseift. Man arbeitet in analoger Weise auf und erhält 2,203 g (79,4 % der Theorie) der Titelverbindung.

| | | | | | | |
|---|---|---|---|---|---|---|
| Analyse: $C_{18}H_{14}F_4N_2O_7S$ (478,38) | | | | | | |
| C 45,19 | H 2,95 | F 15,88 | N 5,86 | O 23,41 | S 6,70 | Ber. |
| C 45,27 | H 3,02 | F 15,79 | N 5,90 | O - | S 6,73 | Gef. |

**Beispiel 41**

2-{4-[N-(4-Fluor-2-trifluormethylphenyl)-sulfamoyl]-benzoylimino}-diessigsäure

a) **2-{4-[N-(4-Fluor-2-trifluormethylphenyl)-sulfamoyl]-benzoylimino}-diessigsäure-diethylester**

Unter den Bedingungen des Beispiels 2a werden 3,633 g (10 mmol) 4-[N-(4-Fluor-2-trifluormethylphenyl)-sulfamoyl]-benzoesäure, 2,257 g (10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,217 g (78,9 % der Theorie)

| | | | | | | |
|---|---|---|---|---|---|---|
| Analyse: $C_{22}H_{22}F_4N_2O_7S$ (534,48) | | | | | | |
| C 49,44 | H 4,15 | F 14,22 | N 5,24 | O 20,95 | S 6,00 | Ber. |
| C 49,52 | H 4,21 | F 14,16 | N 5,28 | O - | S 5,89 | Gef. |

b) **2-{4-[N-(4-Fluor-2-trifluormethylphenyl)-sulfamoyl]-benzoylimino}-diessigsäure**

Unter den Bedingungen des Beispiels 2b werden 2,672 g (5 mmol) des unter Beispiel 41a hergestellten Esters mit 6 ml (12 mmol) 2N Natronlauge in 25 ml Dioxan verseift. Man arbeitet in analoger Weise auf und erhält 1,875 g (78,4 % der Theorie) der Titelverbindung.

| | | | | | | |
|---|---|---|---|---|---|---|
| Analyse: $C_{18}H_{14}F_4N_2O_7S$ (478,38) | | | | | | |
| C 45,19 | H 2,95 | F 15,88 | N 5,86 | O 23,41 | S 6,70 | Ber. |
| C 45,28 | H 3,02 | F 15,80 | N 5,91 | O - | S 6,74 | Gef. |

**Beispiel 42**

2-{2-[N-(4-Fluor-3-trifluormethylphenyl)-sulfamoyl]-benzoylimino}-diessigsäure

a) **2-{2-[N-(4-Fluor-3-trifluormethylphenyl)-sulfamoyl]-benzoylimino}-diessigsäure-diethylester**

Unter den Bedingungen des Beispiels 2a werden 3,633 g (10 mmol) 2-[N-(4-Fluor-3-trifluormethylphenyl)-sulfamoyl]-benzoesäure, 2,257 g (10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird an Kieselgel chromatographiert. Die Titelverbindung wird mit Essigester eluiert.
Ausbeute: 4,287 g (80,2 % der Theorie)

| | | | | | | |
|---|---|---|---|---|---|---|
| Analyse: $C_{22}H_{22}F_4N_2O_7S$ (534,48) | | | | | | |
| C 49,44 | H 4,15 | F 14,22 | N 5,24 | O 20,95 | S 6,00 | Ber. |
| C 49,36 | H 4,22 | F 14,09 | N 5,31 | O - | S 5,89 | Gef. |

b) **2-{2-[N-(4-Fluor-3-trifluormethylphenyl)-sulfamoyl]-benzoylimino}-diessigsäure**

Unter den Bedingungen des Beispiels 2b werden 3,26 g (6,1 mmol) des unter Beispiel 42a hergestellten Esters mit 7 ml (14 mmol) 2N Natronlauge in 25 ml Dioxan verseift. Man arbeitet in analoger Weise auf und erhält 2,285 g (78,3 % der Theorie) der Titelverbindung.

Analyse: $C_{18}H_{14}F_4N_2O_7S$ (478,38)

| | | | | | | |
|---|---|---|---|---|---|---|
| C 45,19 | H 2,95 | F 15,88 | N 5,86 | O 23,41 | S 6,70 | Ber. |
| C 45,30 | H 3,04 | F 15,79 | N 5,82 | O - | S 6,73 | Gef. |

**Beispiel 43**

2-{3-[N-(4-Fluor-2-trifluormethylphenyl)-sulfamoyl]-propionylamino}-essigsäure

a) **2-{3-[N-(4-Fluor-2-trifluormethylphenyl)-sulfamoyl]-propionylamino}-essigsäureethylester**

Unter den Bedingungen des Beispiels 2a werden 3,152 g (10 mmol) 3-[N-(4-Fluor-2-trifluormethylphenyl)-sulfamoyl]-propionsäure, 1,396 g (10 mmol) Glycinethylester Hydrochlorid, 1,531 g (10 mmol) Hydroxybenztriazol Hydrat, sowie 1,012 9 (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,179 g (79,4 % der Theorie)

Analyse: $C_{14}H_{16}F_4N_2O_5S$ (400,35)

| | | | | | | |
|---|---|---|---|---|---|---|
| C 42,00 | H 4,03 | F 18,98 | N 7,00 | O 19,98 | S 8,01 | Ber. |
| C 41,93 | H 4,11 | F 18,91 | N 7,10 | O - | S 7,89 | Gef. |

b) **2-{3-[N-(4-Fluor-2-trifluormethylphenyl)-sulfamoyl]-propionylamino}-essigsäure**

Unter den Bedingungen des Beispiels 2b werden 3,203 g (8 mmol) des unter Beispiel 43a hergestellten Esters mit 9 ml (18 mmol) 2N Natronlauge in 35 ml Dioxan verseift. Es wird in analoger Weise zu Beispiel 2b aufgearbeitet und erhält 2,41 g (80,9 % der Theorie) der Titelverbindung.

Analyse: $C_{12}H_{12}F_4N_2O_5S$ (372,30)

| | | | | | | |
|---|---|---|---|---|---|---|
| C 38,71 | H 3,25 | F 20,41 | N 7,52 | O 21,49 | S 8,61 | Ber. |
| C 38,78 | H 3,30 | F 20,35 | N 7,60 | O - | S 8,58 | Gef. |

**Beispiel 44**

2-{3-[N-(4-Fluor-2-trifluormethylphenyl)-sulfamoyl]-propionylamino}-2-methylessigsäure

a) **2-{3-[N-(4-Fluor-2-trifluormethylphenyl)-sulfamoyl]-propionylamino}-2-methyl-essigsäureethylester**

Unter den Bedingungen des Beispiels 2a werden 3,152 g (10 mmol) 3-[N-(4-Fluor-2-trifluormethylphenyl)-sulfamoyl]-propionsäure, 1,536 g (10 mmol) L-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.

Ausbeute: 3,245 g (78,3 % der Theorie)

Analyse: $C_{15}H_{18}F_4N_2O_5S$ (414,37)

| | | | | | | |
|---|---|---|---|---|---|---|
| C 43,48 | H 4,38 | F 18,34 | N 6,76 | O 19,31 | S 7,74 | Ber. |
| C 43,51 | H 4,46 | F 18,30 | N 6,82 | O - | S 7,67 | Gef. |

b) **2-{3-[N-(4-Fluor-2-trifluormethylphenyl)-sulfamoyl]-propionylamino}-2-methylessigsäure**

Unter den Bedingungen des Beispiels 2b werden 3,108 g (7,5 mmol) des unter Beispiel 44a hergestellten Esters mit 9 ml (18 mmol) 2N Natronlauge in 35 ml Dioxan verseift. Es wird in analoger Weise zu Beispiel 2b aufgearbeitet. Man erhält 2,24 g (77,3 % der Theorie) der Titelverbindung.

Analyse: $C_{13}H_{14}F_4N_2O_5S$ (386,32)

| | | | | | | |
|---|---|---|---|---|---|---|
| C 40,42 | H 3,65 | F 19,67 | N 7,25 | O 20,71 | S 8,30 | Ber. |
| C 40,45 | H 3,70 | F 19,62 | N 7,31 | O - | S 8,24 | Gef. |

**Beispiel 45**

2-{3-[N-(4-Fluor-2-trifluormethylphenyl)-sulfamoyl]-propionylamino}-2-hydroxymethyl-essigsäure

a) **2-{3-[N-(4-Fluor-2-trifluormethylphenyl)-sulfamoyl]-propionylamino}-2-hydroxymethyl-essigsäuremethylester**

Unter den Bedingungen des Beispiels 2a werden 3,152 g (10 mmol) 3-[N-(4-Fluor-2-trifluormethylphenyl)-sulfamoyl]-propionsäure, 1,556 g (10 mmol) L-Serinmethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,372 g (81,0 % der Theorie)

Analyse: $C_{14}H_{16}F_4N_2O_6S$ (416,35)

| | | | | | | |
|---|---|---|---|---|---|---|
| C 40,39 | H 3,87 | F 18,25 | N 6,73 | O 23,06 | S 7,70 | Ber. |
| C 40,30 | H 4,01 | F 18,20 | N 6,67 | O - | S 7,63 | Gef. |

b) **2-{3-[N-(4-Fluor-2-trifluormethylphenyl)-sulfamoyl]-propionylamino}-2-hydroxymethyl-essigsäure**

Unter den Bedingungen des Beispiels 4b werden 2,498 g (6 mmol) des unter Beispiel 45a hergestellten Esters mit 7 ml (14 mmol) 2N Bariumhydroxidlösung in 25 ml Dioxan verseift. Es wird in analoger Weise zu Beispiel 4b aufgearbeitet. Man erhält 1,912 g (79,2 % der Theorie) der Titelverbindung.

Analyse: $C_{13}H_{14}F_4N_2O_6S$ (402,32)

| | | | | | | |
|---|---|---|---|---|---|---|
| C 38,81 | H 3,51 | F 18,89 | N 6,96 | O 23,86 | S 7,97 | Ber. |
| C 38,90 | H 3,59 | F 18,92 | N 7,02 | O - | S 7,93 | Gef. |

**Beispiel 46**

2-{3-[N-(4-Fluor-2-trifluormethylphenyl)-sulfamoyl]-propionylamino}-2-(1-hydroxyethyl)-essigsäure

a) **2-{3-[N-(4-Fluor-2-trifluormethylphenyl)-sulfamoyl]-propionylamino]-2-(1-hydroxyethyl)-essigsäureethylester**

Unter den Bedingungen des Beispiels 2a werden 3,152 g (10 mmol) 3-[N-(4-Fluor-2-trifluormethylphenyl)-sulfamoyl]-propionsäure, 1,736 g (10 mmol) L-Threoninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,484 g (78,4 % der Theorie)

Analyse: $C_{16}H_{20}F_4N_2O_6S$ (444,40)

| | | | | | | |
|---|---|---|---|---|---|---|
| C 43,24 | H 4,54 | F 17,10 | N 6,30 | O 21,60 | S 7,22 | Ber. |
| C 43,16 | H 4,59 | F 17,04 | N 6,35 | O - | S 7,26 | Gef. |

b) **2-{3-[N-(4-Fluor-2-trifluormethylphenyl)-sulfamoyl]-propionylamino}-2-(1-hydroxyethyl)-essigsäure**

Unter den Bedingungen des Beispiels 4b werden 2,666 g (6 mmol) des unter Beispiel 46a hergestellten Esters mit 6,5 ml (13 mmol) 2N Bariumhydroxidlösung in 30 ml Dioxan verseift. Es wird in analoger Weise zu Beispiel 4b aufgearbeitet. Man erhält 1,871 g (74,9 % der Theorie) der Titelverbindung.

Analyse: $C_{14}H_{16}F_4N_2O_6S$ (416,35)

| | | | | | | |
|---|---|---|---|---|---|---|
| C 40,39 | H 3,87 | F 18,25 | N 6,73 | O 23,06 | S 7,70 | Ber. |
| C 40,29 | H 3,83 | F 18,18 | N 6,66 | O - | S 7,61 | Gef. |

**Beispiel 47**

3-{3-[N-(4-Fluor-2-trifluormethylphenyl)-sulfamoyl]-propionylamino}-3-carboxypropionsäure

a) **3-{3-[N-(4-Fluor-2-trifluormethylphenyl)-sulfamoyl]-propionylamino}-3-methoxycarbonyl-propionsäuremethylester**

Unter den Bedingungen des Beispiels 2a werden 3,152 g (10 mmol) 3-[N-(4-Fluor-2-trifluormethylphenyl)-sulfamoyl]-propionsäure, 1,836 g (10 mmol) L-Asparaginsäuredimethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,557 g (77,6 % der Theorie)

Analyse: $C_{16}H_{18}F_4N_2O_7S$ (458,38)

| | | | | | | |
|---|---|---|---|---|---|---|
| C 41,93 | H 3,96 | F 16,58 | N 6,11 | O 24,43 | S 7,00 | Ber. |
| C 41,98 | H 4,03 | F 16,62 | N 6,08 | O - | S 6,94 | Gef. |

b) **3-{3-[N-(4-Fluor-2-trifluormethylphenyl)-sulfamoyl]-propionylamino}-3-carboxy-propionsäure**

Unter den Bedingungen des Beispiels 2b werden 2,750 g (6 mmol) des unter Beispiel 47a hergestellten Esters mit 7 ml (14 mmol) 2N Natronlauge in 25 ml Dioxan verseift. Es wird in analoger Weise zu Beispiel 2b aufgearbeitet. Man erhält 2,102 g (81,4 % der Theorie) der Titelverbindung.

Analyse: $C_{14}H_{14}F_4N_2O_7S$ (430,33)

| | | | | | | |
|---|---|---|---|---|---|---|
| C 39,08 | H 3,28 | F 17,66 | N 6,51 | O 26,03 | S 7,45 | Ber. |
| C 39,00 | H 3,35 | F 17,61 | N 6,58 | O - | S 7,50 | Gef. |

**Beispiel 48**

3-{3-[N-(4-Fluor-2-trifluormethylphenyl)-sulfamoyl]-propionylamino}-propionsäure

a) **3-{3-[N-(4-Fluor-2-trifluormethylphenyl)-sulfamoyl]-propionylamino}-propionsäure-ethylester**

Unter den Bedingungen des Beispiels 2a werden 3,152 g (10 mmol) 3-[N-(4-Fluor-2-trifluormethylphenyl)-sulfamoyl]-propionsäure, 1,536 g (10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,282 g (79,2 % der Theorie)

Analyse: $C_{15}H_{18}F_4N_2O_5S$ (414,37)

| | | | | | | |
|---|---|---|---|---|---|---|
| C 43,48 | H 4,38 | F 18,34 | N 6,76 | O 19,31 | S 7,74 | Ber. |
| C 43,44 | H 4,47 | F 18,38 | N 6,70 | O - | S 7,69 | Gef. |

b) **3-{3-[N-(4-Fluor-2-trifluormethylphenyl)-sulfamoyl]-propionylamino}-propionsäure**

Unter den Bedingungen des Beispiels 2b werden 2,196 g (5,3 mmol) des unter Beispiel 48a hergestellten Esters mit 6 ml (12 mmol) 2N Natronlauge in 25 ml Dioxan verseift. Es wird in analoger Weise zu Beispiel 2b aufgearbeitet. Man erhält 1,609g(78,6 % der Theorie) der Titelverbindung.

Analyse: $C_{13}H_{14}F_4N_2O_5S$ (386,32)

| | | | | | | |
|---|---|---|---|---|---|---|
| C 40,42 | H 3,65 | F 19,67 | N 7,25 | O 20,71 | S 8,30 | Ber. |
| C 40,40 | H 3,71 | F 19,70 | N 7,20 | O - | S 8,28 | Gef. |

pKa (37 °C): 6,91    ppm/pH: 5,21    $LD_{50}$ [mmol/kg], Maus: 4

**Beispiel 49**

2-{3-[N-(4-Fluor-2-trifluormethylphenyl)-sulfamoyl]-propionylimino}-diessigsäure

a) **2-{3-[N-(4-Fluor-2-trifluormethylphenyl)-sulfamoyl]-propionylimino}-diessigsäure-diethylester**

Unter den Bedingungen des Beispiels 2a werden 3,152 g (10 mmol) 3-[N-(4-Fluor-2-trifluormethylphenyl)-sulfamoyl]-propionsäure, 2,257 g (10 mmol) Iminodiessigsäurediethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie

an Kieselgel gereinigt. Die Titelverbindung wird mit Essigester eluiert.
Ausbeute: 3,741 g (76,9 % der Theorie)

Analyse: $C_{18}H_{22}F_4N_2O_7S$ (486,44)

| | | | | | | | |
|---|---|---|---|---|---|---|---|
| C 44,45 | H 4,56 | F 15,62 | N 5,76 | O 23,02 | S 6,59 | Ber. |
| C 44,39 | H 4,51 | F 15,56 | N 5,71 | O - | S 6,52 | Gef. |

b) **2-{3-[N-(4-Fluor-2-trifluormethylphenyl)-sulfamoyl]-propionylimino}-diessigsäure**

Unter den Bedingungen des Beispiels 2b werden 2,432 g (5 mmol) des unter Beispiel 49a hergestellten Esters mit 8 ml (16 mmol) 2N Natronlauge in 30 ml Dioxan verseift. Es wird in analoger Weise zu Beispiel 2b aufgearbeitet. Man erhält 1,683 g (78,2 % der Theorie) der Titelverbindung.

Analyse: $C_{14}H_{14}F_4N_2O_7S$ (430,33)

| | | | | | | |
|---|---|---|---|---|---|---|
| C 39,08 | H 3,28 | F 17,66 | N 6,51 | O 26,03 | S 7,45 | Ber. |
| C 39,02 | H 3,33 | F 17,61 | N 6,46 | O - | S 7,51 | Gef. |

**Beispiel 50**

2-{4-[N-(4-Fluor-3-trifluormethylphenyl)-sulfamoylmethyl]-benzoylamino}-essigsäure

a) **2-{4-[N-(4-Fluor-3-trifluormethylphenyl)-sulfamoylmethyl] benzoylamino}-essigsäureethylester**

Unter den Bedingungen des Beispiels 2a werden 3,773 g (10 mmol) 4-[N-(4-Fluor-3-trifluormethylphenyl)-sulfamoylmethyl]-benzoesäure, 1,396 g (10 mmol) Glycinethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,625 g (78,4 % der Theorie)

Analyse: $C_{19}H_{18}F_4N_2O_5S$ (462,42)

| | | | | | | |
|---|---|---|---|---|---|---|
| C 49,35 | H 3,92 | F 16,43 | N 6,06 | O 17,30 | S 6,93 | Ber. |
| C 49,42 | H 3,99 | F 16,37 | N 6,02 | O - | S 6,88 | Gef. |

b) **2-{4-[N-(4-Fluor-3-trifluormethylphenyl)-sulfamoylmethyl] benzoylamino}-essigsäure**

Unter den Bedingungen des Beispiels 2b werden 1,850 g (4 mmol) des unter Beispiel 50a hergestellten Esters mit 5 ml (10 mmol) 2N Natronlauge in 20 ml Dioxan verseift. Es wird in analoger Weise zu Beispiel 2b aufgearbeitet. Man erhält 1,395 g (80,3 % der Theorie) der Titelverbindung.

Analyse: $C_{17}H_{14}F_4N_2O_5S$ (434,37)

| | | | | | | |
|---|---|---|---|---|---|---|
| C 47,01 | H 3,25 | F 17,49 | N 6,45 | O 18,42 | S 7,38 | Ber. |
| C 47,07 | H 3,29 | F 17,42 | N 6,48 | O - | S 7,34 | Gef. |

**Beispiel 51**

2-{4-[N-(4-Fluor-3-trifluormethylphenyl)-sulfamoylmethyl] benzoylamino}-3-hydroxymethyl-essigsäure

a) **2-{4-[N-(4-Fluor-3-trifluormethylphenyl)-sulfamoylmethyl]-benzoylamino}-2-hydroxymethyl-essigsäuremethylester**

Unter den Bedingungen des Beispiels 2a werden 3,773 g (10 mmol) 4-[N-(4-Fluor-3-trifluormethylphenyl)-sulfamoylmethyl]-benzoesäure, 1,556 g (10 mmol) L-Serinmethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,88 g (81,1 % der Theorie)

Analyse: $C_{19}H_{18}F_4N_2O_6S$ (478,42)

| | | | | | | |
|---|---|---|---|---|---|---|
| C 47,70 | H 3,79 | F 15,88 | N 5,86 | O 20,07 | S 6,70 | Ber. |
| C 47,64 | H 3,83 | F 15,83 | N 5,90 | O - | S 6,65 | Gef. |

b) **2-{4-[N-(4-Fluor-3-trifluormethylphenyl)-sulfamoylmethyl]-benzoylamino}-2-hydroxymethyl-essigsäure**

Unter den Bedingungen des Beispiels 4b werden 3,588 g (7,5 mmol) des unter Beispiel 51a hergestellten Esters mit 8 ml (16 mmol) 2N Bariumhydroxidlösung in 40 ml Dioxan verseift. Es wird in analoger Weise zu Beispiel 4b aufgearbeitet. Man erhält 2,731 g (78,4 % der Theorie) der Titelverbindung.

Analyse: $C_{18}H_{16}F_4N_2O_6S$ (464,39)

| | | | | | | |
|---|---|---|---|---|---|---|
| C 46,56 | H 3,47 | F 16,36 | N 6,03 | O 20,07 | S 6,90 | Ber. |
| C 46,51 | H 3,44 | F 16,32 | N 6,08 | O - | S 6,85 | Gef. |

**Beispiel 52**

3-{4-[N-(4-Fluor-3-trifluormethylphenyl)-sulfamoylmethyl]-benzoylamino}-propionsäure

a) **3-{4-[N-(4-Fluor-3-trifluormethylphenyl)-sulfamoylmethyl]-benzoylamino}-propionsäure-ethylester**

Unter den Bedingungen des Beispiels 2a werden 3,773 g (10 mmol) 4-[N-(4-Fluor-3-trifluormethylphenyl)-sulfamoylmethyl]-benzoesäure, 1,536 g(10 mmol) β-Alaninethylester Hydrochlorid, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird durch Säulenchromatographie an Kieselgel gereinigt. Die Titelverbindung wird mit Essigester eluiert.
Ausbeute: 3,187 g (76,9 % der Theorie)

Analyse: $C_{15}H_{18}F_4N_2O_5S$ (414,37)

| | | | | | | |
|---|---|---|---|---|---|---|
| C 43,48 | H 4,38 | F 18,34 | N 6,76 | O 19,31 | S 7,74 | Ber. |
| C 43,52 | H 4,44 | F 18,30 | N 6,72 | O - | S 7,70 | Gef. |

b) **3-{4-[N-(4-Fluor-3-trifluormethylphenyl)-sulfamoylmethyl]-benzoylamino}-propionsäure**

Unter den Bedingungen des Beispiels 2b werden 2,486 g (6 mmol) des unter Beispiel 52a hergestellten Esters mit 7 ml (14 mmol) 2N Natronlauge in 35 ml Dioxan verseift. Es wird in analoger Weise zu Beispiel 2b aufgearbeitet. Man erhält 1,806 g (77,9 % der Theorie) der Titelverbindung.

Analyse: $C_{13}H_{14}F_4N_2O_5S$ (386,32)

| | | | | | | |
|---|---|---|---|---|---|---|
| C 40,42 | H 3,65 | F 19,67 | N 7,25 | O 20,71 | S 8,30 | Ber. |
| C 40,38 | H 3,66 | F 19,60 | N 7,22 | O - | S 8,27 | Gef. |

**Beispiel 53**

2-{2-[N-(4-Fluor-2-trifluormethylphenyl)-sulfamoyl]-acetylamino}-ethansulfonsäure

Unter den Bedingungen des Beispiels 2a werden 3,012 g (10 mmol) 2- N-(4-Fluor-2-trifluormethylphenyl)-sulfamoyl]-essigsäure, 1,252 g (10 mmol) 2-Aminoethansulfonsäure, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird in destilliertem Wasser gelöst, mit Essigester extrahiert und durch Behandeln mit saurem Kationenaustauscher in die frei Säure überführt. Man erhält die Titelverbindung durch Gefriertrocknung der wässrigen Lösung.
Ausbeute: 2,952 g (72,3 % der Theorie)

Analyse: $C_{11}H_{12}F_4N_2O_6S_2$ (408,35)

| | | | | | | |
|---|---|---|---|---|---|---|
| C 32,36 | H 2,96 | F 18,61 | N 6,86 | O 23,51 | S 15,70 | Ber. |
| C 32,28 | H 3,04 | F 18,55 | N 6,82 | O - | S 15,68 | Gef. |

**Beispiel 54**

4-{2-[N-(4-Fluor-2-trifluormethylphenyl)-sulfamoyl]-acetylamino}-benzolsulfonsäure

Unter den Bedingungen des Beispiels 2a werden 3,012 g (10 mmol) 2-[N- (4-Fluor-2-trifluormethylphenyl-sulfamoyl]-essigsäure, 1,732 g (10 mmol) 4-Amino-benzolsulfonsäure, 1,531 g (10 mmol) Hydroxybenztriazol Hydrat, sowie 1,012 g (10 mmol) Triethylamin und 2,063 g (10 mmol) Dicyclohexylcarbodiimid umgesetzt und in analoger Weise aufgearbeitet. Das Rohprodukt wird in destilliertem Wasser gelöst, mit Essigester extrahiert und durch Behandeln mit saurem Kationenaustauscher in die freie Säure überführt. Man erhält die Titelverbindung durch Gefriertrocknung der wässrigen Lösung.
Ausbeute: 3,706 g (81,2 % der Theorie)

Analyse: $C_{15}H_{12}F_4N_2O_6S_2$ (456,39)

| | | | | | | |
|---|---|---|---|---|---|---|
| C 39,48 | H 2,65 | F 16,65 | N 6,14 | O 21,03 | S 14,05 | Ber. |
| C 39,52 | H 2,72 | F 16,60 | N 6,18 | O - | S 14,00 | Gef. |

**Beispiel 55**

2-{2-[N-(4-Fluor-2-trifluormethylphenyl)-sulfamoyl]-acetylimino}-diessigsäuremono-(N-methyl-N-2,3-dihydroxypropyl)-amid

In 120 ml Dimethylformamid werden 4,163 g (10 mmol) der unter Beispiel 10b hergestellten Diessigsäure mit 2,476 g (12 mmol) Dicyclohexylcarbodiimid über Nacht bei Raumtemperatur gerührt. Dann tropft man unter Rühren 2,103 g (20 mmol) N-Methyl-2,3-dihydroxy-propylamin in 20 ml Dimethylformamid dazu und läßt 5 Stunden rühren. Man engt im Vakuum zur Trockene ein, nimmt in destilliertem Wasser auf und filtriert vom Harnstoff ab. Die Lösung wird mit Essigester extrahiert. Durch Zugabe von Kationenaustauscher in der $H^+$-Form wird die Säure aus dem Salz freigesetzt. Man filtriert vom Austauscher und gewinnt die Titelverbindung durch Gefriertrocknung der wässrigen Lösung.
Ausbeute: 4,239 g (84,2 % der Theorie)

Analyse: $C_{17}H_{21}F_4N_3O_8S$ (503,45)

| | | | | | | |
|---|---|---|---|---|---|---|
| C 40,56 | H 4,21 | F 15,09 | N 8,35 | O 25,42 | S 6,37 | Ber. |
| C 40,50 | H 4,28 | F 15,12 | N 8,41 | O - | S 6,31 | Gef. |

**Beispiel 56**

Herstellung einer Lösung von 2-{2-[N-(4-Fluor-3-trifluormethylphenyl)-sulfamoyl]-acetylamino}-essigsäure

179,13 g (0,5 Mol) der in Beispiel 2b beschriebenen Verbindung werden in 500 ml Wasser p.i. gegeben. Durch Zugabe von 5-normaler Natronlauge wird eine Lösung hergestellt, die anschließend mit 0,1 normaler Natronlauge auf pH 7,2 eingestellt wird. Nach Zugabe von 100 mg $CaNa_2$ EDTA wird mit Wasser p.i. auf 1000 ml aufgefüllt. Die Lösung wird sterilfiltriert und in Multivials und/oder Ampullen abgefüllt.

**Beispiel 57**

Herstellung einer Lösung von 2-{2-[N-(4-Fluor-2-trifluormethylphenyl)-sulfamoyl]-acetylamino}-ethansulfonsäure

204,18 g (0,5 Mol) der in Beispiel 53 beschriebenen Verbindung werden in 500 ml Wasser p.i. gegeben. Durch Zugabe von 5-normaler Natronlauge wird eine Lösung hergestellt, die anschließend mit 0,1 normaler Natronlauge auf pH 7,2 eingestellt wird. Nach Zugabe von 100 mg $CaNa_2$ EDTA wird mit Wasser p.i. auf 1000 ml aufgefüllt. Die Lösung wird sterilfiltriert und in Multivials und/oder Ampullen abgefüllt.

**Darstellung der Ausgangsverbindungen:**

3-[N-(4-Fluor-2-trifluormethylphenyl)-sulfamoyl]-propionsäure

a) **3-[N-(4-Fluor-2-trifluormethylphenyl)-sulfamoyl]-propionsäuremethylester**

In 100 ml Pyridin werden 8,96 g (50 mmol) 4-Fluor-2-trifluormethyl-anilin gelöst und in der Kälte unter Rühren mit 9,52 g (50 mmol) 3-Chlorsulfonylpropionsäure-methylester (98,6 %) versetzt. Man läßt 20 Minuten ruhren, erwärmt 4 Stunden auf 40°C und zieht das Pyridin im Vakuum ab. Der Rückstand wird zwischen Dichlorethan und 4N Salzsäure verteilt. Die organische Lösung wird mit Wasser gewaschen, über Natriumsulfat getrocknet und im Vakuum zur Trockene eingeengt. Der Rückstand wird durch Säulenchromatographie an Kieselgel gereinigt. Die Titelverbindung wird mit Essigester eluiert.
Ausbeute: 11,920 g (72,4 % der Theorie)

Analyse: $C_{11}H_{11}F_4NO_4S$ (329,27)

| | | | | | | |
|---|---|---|---|---|---|---|
| C 40,13 | H 3,37 | F 23,08 | N 4,25 | O 19,44 | S 9,74 | Ber. |
| C 40,22 | H 3,44 | F 23,03 | N 4,21 | O - | S 9,70 | Gef. |

b) **3-[N-(4-Fluor-2-trifluormethylphenyl)-sulfamoyl]-propionsäure**

In 50 ml Dioxan werden 3,293 g (10 mmol) des unter a) hergestellten Esters gelöst und mit 10 ml (20 mmol) 2N Natronlauge versetzt. Man rührt bei 50°C bis kein Ausgangsmaterial mehr nachweisbar ist. Die Losung wird mit Salzsäure auf einen pH-Wert von 4 eingestellt und im Vakuum zur Trockene eingeengt. Man verteilt den Ruckstand zwischen Dichlormethan und angesäuertem Wasser . Die organische Lösung wird mit Wasser gewaschen, über Natriumsulfat getrocknet und im Vakuum zur Trockene eingeengt. Man erhält so 2,572 g (81,6 % der Theorie) der Titelverbindung.

Analyse: $C_{10}H_9F_4NO_4S$ (315,24)

| | | | | | | |
|---|---|---|---|---|---|---|
| C 38,10 | H 2,88 | F 24,11 | N 4,44 | O 20,30 | S 10,17 | Ber. |
| C 38,15 | H 2,95 | F 24,03 | N 4,48 | O - | S 10,09 | Gef. |

**2-[N-(4-Fluor-2-trifluormethylphenyl)-sulfamoyl]-benzoesäuremethylester**

In 250 ml Dichlormethan werden 9,38 g (52,37 mmol) 4-Fluor-2-trifluormethyl-anilin und 6 ml (14,3 mmol) Pyridin vorgelegt. Man kühlt mit Eiswasser und gibt unter Rühren und Ausschluß von Feuchtigkeit 13,52 g (57,62 mmol) 2-Chlorsulfonyl-benzoesäuremethylester in kleinen Anteilen dazu. Man läßt über Nacht rühren, wäscht mit 2N Salzsäure und Wasser, trocknet die Dichlormethanlösung über Natriumsulfat und engt im Vakuum zur Trockene ein. Der Rückstand wird aus Essigester/Hexan kristallisiert. Die Titelverbindung schmilzt bei 143-45°C.
Ausbeute: 10,28 g (52,0 % der Theorie)

**2-[N-(4-Fluor-2-trifluormethylphenyl)-sulfamoyl]-benzoesäure**

In 60 ml Dioxan werden 6,25 g (16,56 mmol) 2-[N-(4-Fluor-2-trifluormethylphenyl)-sulfamoyl]-benzoesäuremethylester gelöst und mit 25 ml (50 mmol) 2N Natronlauge versetzt. Man erwärmt auf dem Wasserbad, bis kein Ausgangsmaterial mehr vorhanden ist. Die Lösung wird dann im Vakuum zur Trockene eingeengt, mit 2N Salzsäure versetzt und dar ausgefallene Material wird in Essigester gelöst. Man trocknet die organische Lösung über Natriumsulfat, engt zur Trockene ein und kristallisiert den Rückstand aus Essigester/Hexan. Die Titelverbindung schmilzt bei 148°C.
Ausbeute: 5,45 g (90,5 % der Theorie)

2-[N-(4-Fluor-3-trifluormethylphenyl)-sulfamoyl]-benzoesäuremethylester

In 150 ml Dichlormethan werden 9,05 g (50 mmol) 4-Fluor-3-trifluormethyl-anilin (99 %) und 5 ml (62 mmol) Pyridin gelöst. Man kühlt auf -5°C und gibt dann unter Rühren und Feuchtigkeitsausschluß 13,03 g (50 mmol) 2-Chlorsulfonyl-benzoesäuremethylester (90 %) in kleinen Anteilen dazu. Man läßt 2 Tage bei Raumtemperatur rühren, wäscht mit 2N Salzsäure und Wasser, trocknet die Dichlormethanlösung über Natriumsulfat und engt im Vakuum zur Trockene ein. Der Rückstand wird aus Essigester/Hexan kristallisiert. Die Titelverbindung schmilzt bei 99-101°C.
Ausbeute: 12,093 g (64,1 % der Theorie)

2-[N-(4-Fluor-3-trifluormethylphenyl)-sulfamoyl]-benzoesäure

In 70 ml Dioxan werden 10,37 g (27,5 mmol) 2-[N-(4-Fluor-3-trifluormethylphenyl)-sulfamoyl]-benzoesäuremethylester gelöst und wie in vorstehendem Beispiel beschrieben mit 40 ml (80 mmol) 2N Natronlauge verseift. Man arbeitet in analoger Weise auf und erhält nach Kristallisation aus Essigester/Hexan die Titelverbindung vom Schmelzpunkt 164-66°C.
Ausbeute: 9,32 g (93,3 % der Theorie)

4-[N-(4-Fluor-3-trifluormethylphenyl)-sulfamoyl]-benzoesäure

In 250 ml Dichlormethan werden 10 g (55,8 mmol) 4-Fluor-3-trifluormethyl-anilin und 6 ml (74,3 mmol) Pyridin gelöst. Man kühlt mit Eiswasser und gibt unter Rühren und Feuchtigkeitsausschluß 12,7 g (57,6 mmol) 4-Chlorsulfonylbenzoesäure anteilsweise dazu. Man läßt über Nacht rühren, versetzt den Brei mit 75 ml 2N Natronlauge und trennt die organische Phase ab. Die alkalische Lösung wird mit Dichlormethan gewaschen und dann mit Salzsäure auf einen pH-Wert von 2 eingestellt. Das Produkt wird in Essigester aufgenommen. Die Lösung wird mit Wasser gewaschen, über Natriumsulfat getrocknet und zum Trockene eingeengt. Die Titelverbindung schmilzt nach Kristallisation aus Ethanol bei 243-45°C.
Ausbeute: 15,54 g (76,7 % der Theorie)

2-[N-(4-Fluor-3-trifluormethylphenyl)-sulfamoyl]-essigsäuremethylester

In 350 ml Dichlormethan werden 13,02 g (72,70 mmol) 4-Fluor-3-trifluormethyl-anilin und 6,79 ml (84,12

mmol) Pyridin gelöst. Man kühlt auf 0°C und tropft dann unter Rühren und Feuchtigkeitsausschluß 13,2 g (72,70 mmol) 2-Chlorsulfonyl-essigsäuremethylester (95.3 %) in 50 ml Dichlormethan gelöst dazu. Nach Rühren über Nacht wird mit 2N Salzsäure und Wasser gewaschen. Die Dichlormethanlösung wird über Natriumsulfat getrocknet, im Vakuum zur Trockene eingeengt. Der Rückstand wird aus Diethylether/Hexan kristallisiert. Die Titelverbindung schmilzt bei 111-13°C.
Ausbeute: 19,86 g (86,7 % der Theorie)

2-[N-(4-Fluor-3-trifluormethylphenyl)-sulfamoyl]-essigsäure

In 110 ml Dioxan werden 12,79 g (40,57 mmol) 2-[N-(4-Fluor-3-trifluormethylphenyl)-sulfamoyl]-essigsäuremethylester gelöst und mit 59 ml (118 mmol) 2N Natriumlauge versetzt. Es erfolgte leichte Erwärmung, und nach 15 Minuten war kein Ausgangsmaterial mehr vorhanden. Man engte im Vakuum zur Trockene ein, versetzte den Rückstand mit 2N Salzsäure und nahm die ausgefallene Säure in Essigester auf. Die organische Lösung wird mit Wasser gewaschen, über Natriumsulfat getrocknet und im Vakuum zur Trockene eingeengt. Der Rückstand wird aus Essigester/Hexan kristallisiert. Die Titelverbindung schmilzt bei 138-40°C.
Ausbeute: 11,17 g (91,4 % der Theorie)

4-[N-(4-Fluor-2-trifluormethlphenyl)sulfamoylmethyl]-benzoesäure-methylester

5.29g (29.5 mmol) 4-Fluor-2-(trifluormethyl)-anilin und 5ml Pyridin werden unter Feuchtigkeitsausschluß in 200ml Dichlormethan gelöst und die Lösung auf 0°C gekühlt. Zu dieser Lösung werden portionsweise 7.5g (29.5 mmol) 4-Chlorsulfonylmethylbenzoesäure-methylester hinzugefügt und 1 Stunde bei 0°C gerührt. Die Reaktionslösung wird mit 2N Salzsäure pyridinfrei gewaschen, zweimal mit Wasser ausgeschüttelt, die organische Phase über Natriumsulfat getrocknet, filtriert und zur Trockne eingedampft. Der Rückstand wird im Vakuum getrocknet. Man erhält 10.27g (26.24 mmol) = 89% d. Th.

| | | | | | | | | | | | | | |
|---|---|---|---|---|---|---|---|---|---|---|---|---|---|
| Analyse | : | $C_{16}H_{13}F_4NO_4S$ | | MG 391.34 | | | | | | | | | |
| berechnet | : | C | 49.11 | H | 3.35 | F | 19.42 | N | 3.58 | O | 16.35 | S | 8.19% |
| gefunden | : | | 49.39 | | 3.23 | | 19.67 | | 3.66 | | | | 8.15% |

4-[N-(4-Fluor-2-trifluormethylphenyl)sulfamolymethyl]-benzoesäure

8.6g (21.98 mmol) 4-[N-(4-Fluor-2-trifluormethylphenyl)sulfamoylmethyl]-benzoesäuremethylester werden in 150ml Dioxan gelöst. bzw. suspendiert. 25m (50 mmol) 2N Natronlauge hinzugefügt und 15 Minuten bei 60°C gerührt. Die Lösung wird dann mit konzentrierter Salzsäure angesäuert, wobei die Säure als Feststoff ausfällt. Sie wird abgesaugt, mit Wasser gewaschen und in 50ml Essigester gelöst. Diese Lösung wird über Natriumsulfat getrocknet, filtriert, eingedampft und der Rückstand im Vakuum getrocknet. Man erhält 7.35g (19.48 mmol) = 88.62%. d. Th.

| | | | | | | | | | | | | | |
|---|---|---|---|---|---|---|---|---|---|---|---|---|---|
| Analyse | : | $C_{15}H_{11}F_4NO_4S$ | | MG 377.31 | | | | | | | | | |
| berechnet | : | C | 47.75 | H | 2.94 | F | 20.14 | N | 3.71 | O | 16.96 | S | 8.5% |
| gefunden | : | | 47.88 | | 3.07 | | 20.25 | | 3.56 | | | | 8.35% |

2-[N-(4-Fluor-2-trifluormethylphenyl)sulfamoyl]-essigsäuremethylester

5g (27.64 mmol) 4-Fluor-2-(trifluormethyl)-anilin werden in 25m trockenem Pyridin gelöst und die Lösung auf 0°C gekühlt. Zur gekühlten Lösung tropft man unter Beibehaltung der Temperatur im Verlaufe von ca. 10 Minuten eine Lösung von 4.82g (27.4 mmol) Chlorsulfonylessigsäuremethylester in 20ml Dichlormethan. Anschließend wird 6 Stunden bei Raumtemperatur gerührt. Die Reaktionslösung wird dann mit 100ml Dichlormethan verdünnt, das Pyridin mit 2N Salzsäure ausgeschüttelt, die organische Phase über Magnesiumsulfat getrocknet, filtriert und zur Trockne eingedampft. Der Rückstand wird aus Diethylether/ Hexan 1 : 1 kristallisiert. Man erhält 7.35g (23.32 mmol) = 84.35% d. Th. Kristallisat. Fp. 84-86 °C.

2-[N-(4-Fluor-2-trifluormethylphenyl)sulfamoyl]-essigsäure

2.2g (6.98 mmol) 2-[N-(4-Fluor-2-trifluormethylphenyl)sulfamoyl]-essigsäuremethylester werden in 20ml Dioxan gelöst, und die Lösung mit 10m (20 mmol) 2N Natronlauge versetzt. Es tritt eine leichte Erwärmung ein und die Verseifung ist nach 10 Minuten vollständig. Die Lösung wird mit 2N Salzsäure unter pH-Kontrolle neutralisiert und im Vakuum schonend zur Trockne eingedampft. Der Rückstand wird mit 30ml Essigester extrahiert, die Lösung einmal mit 10ml Wasser ausgeschüttet, die organische Phase über Magnesiumsulfat getrocknet, filtriert und zur Trockne eingedampft. Man erhält 1.96g (6.51 mmol) = 93.2% d. Th.

| | | | | | | | | | | | | | | |
|---|---|---|---|---|---|---|---|---|---|---|---|---|---|---|
| Analyse | : | $C_9H_7F_4NO_4S$ MG 301.24 | | | | | | | | | | | | |
| berechnet | : | C | 35.89 | H | 2.34 | F | 25.23 | N | 4.65 | O | 21.25 | S | 10.64 | % |
| gefunden | : | | 36.05 | | 2.36 | | 25.45 | | 4.66 | | | | 10.79 | % |

4-[N-(4-Fluor-2-trifluormethylphenyl)sulfamoyl]-benzoesäure

2.21g (10 mmol) 4-Chlorsulfonyl-benzoesäure werden in 30ml Dichlormethan unter Feuchtigkeitsausschluß suspendiert, die Suspension auf -5°C gekühlt und bei dieser Temperatur eine Lösung von 1.79g (10 mmol) 4-Fluor-2-(trifluormethyl)-anilin und 1ml Pyridin in 20ml Dichlormethan zugetropft. Nach beendetem Eintropfen läßt man auf Raumtemperatur kommen und rührt 12 Stunden nach. Der entstandene Niederschlag wird abgesaugt und mit Ether extrahiert. Dieser Etherextrakt und das Filtrat werden zusammen eingedampft und der Rückstand in Dichlorethan gelöst. Die Lösung wird dreimal mit je 10ml gesättigter Natriumhydrogencarbonat-Lösung extrahiert. Die vereinigten wässrigen Extrakte werden mit 2N Salzsäure angesäuert und das dabei ausfallende Produkt abfiltriert. Der Filterrückstand wird im Vakuum bei 50°C getrocknet. Man erhält 3.3g (9.08 mmol) = 90.84% d. Th.

| | | | | | | | | | | | | | | |
|---|---|---|---|---|---|---|---|---|---|---|---|---|---|---|
| Analyse | : | $C_{14}H_9F_4NO_4S$ MG 363.28 | | | | | | | | | | | | |
| berechnet | : | C | 46.29 | H | 2.5 | F | 20.92 | N | 3.86 | O | 17.62 | S | 8.83 | % |
| gefunden | : | | 46.10 | | 2.67 | | 21.16 | | 3.84 | | | | 8.96 | % |

## Patentansprüche

1. Fluorbenzolsulfonamide der allgemeinen Formel I

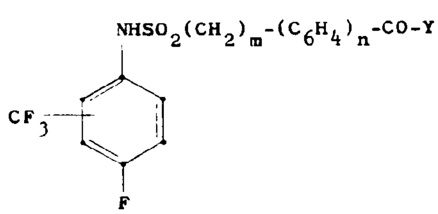

(I),

worin
m für die Ziffern 0, 1, 2, 3 oder 4,
n für die Ziffern 0 oder 1 und
Y für den Rest einer Aminocarbon- oder Aminosulfonsäure
stehen, mit der Maßgabe, daß m und n nicht gleichzeitig für die Ziffer 0 stehen sollen und gewünschtenfalls die Säuregruppen in Form ihrer Amide oder in Form von Salzen mit organischen oder anorganischen Basen vorliegen.

2. Verbindungen nach Anspruch 1, dadurch gekennzeichnet, daß Y für die Reste

$-NH-CH_2COR^1$

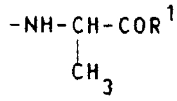 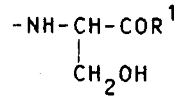 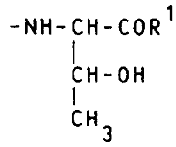

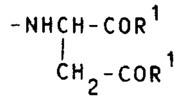

$-NH-CH_2-CH_2-COR^1$

$-N(CH_2COR^1)_2$

$-NH-(CH_2)_m-(C_6H_4)_n-SO_2R^1$

mit $R^1$ in der Bedeutung einer Hydroxygruppe oder eines

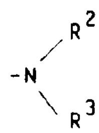

-Restes,
worin
$R^2$ und $R^3$ unabhängig voneinander ein Wasserstoffatom, eine gerade oder verzweigte, gesättigte oder ungesättigte, gegebenenfalls durch 1 bis 5 Hydroxy- oder $C_1$-$C_4$-Alkoxygruppen substituierte Alkylgruppe mit 1 bis 16 Kohlenstoffatomen oder eine Aryl- oder Aralkylgruppe, oder
$R^2$ und $R^3$ gemeinsam mit dem Stickstoffatom einen gegebenenfalls ein weiteres Stickstoff-, Sauerstoff-, Schwefelatom oder eine Carbonylgruppe enthaltenden gesättigten oder ungesättigten Fünf- oder Sechsring bedeuten,
steht.

3. 2-{2-[N-(4-Fluor-2-trifluormethylphenyl)-sulfamoyl]-acetylamino}-essigsäure
2-{2-[N-(4-Fluor-3-trifluormethylphenyl)-sulfamoyl]-acetylamino}-essigsäure
2-{2-[N-(4-Fluor-2-trifluormethylphenyl)-sulfamoyl]-acetylamino}-2-hydroxymethyl-essigsäure
2-{2-[N-(4-Fluor-3-trifluormethylphenyl)-sulfamoyl]-acetylamino}-2-hydroxymethyl-essigsäure
3-{2[N-(4-Fluor-2-trifluormethylphenyl)-sulfamoyl]-acetylamino}-3-carboxy-propionsäure
3-{2-[N-(4-Fluor-3-trifluormethylphenyl)-sulfamoyl]-acetylamino}-3-carboxy-propionsäure
2-{4-[N-(4-Fluor-2-trifluormethylphenyl)-sulfamoyl]-benzoylamino}-essigsäure
2-{4-[N-(4-Fluor-3-trifluormethylphenyl)-sulfamoyl]-benzoylamino}-essigsäure
2-{4-[N-(4-Fluor-2-trifluormethylphenyl)-sulfamoyl]-benzoylamino}-2-hydroxymethyl-essigsäure
2-{4-[N-(4-Fluor-3-trifluormethylphenyl)-sulfamoyl]-benzoylamino}-2-hydroxymethyl-esssigsäure
3-{4-[N-(4-Fluor-2-trifluormethylphenyl)-sulfamoyl]-benzoylamino}-3-carboxy-propionsäure
3-{4-[N-(4-Fluor-3-trifluormethylphenyl)-sulfamoyl]-benzoylamino}-3-carboxy-propionsäure
2-{2-[N-(4-Fluor-2-trifluormethylphenyl)-sulfamoyl]-benzoylamino}-essigsäure
2-{2-[N-(4-Fluor-3-trifluormethylphenyl)-sulfamoyl]-benzoylamino}-essigsäure
2-{2-[N-(4-Fluor-2-trifluormethylphenyl)-sulfamoyl]-benzoylamino}-2-hydroxymethyl-essigsäure
2-{2-[N-(4-Fluor-3-trifluormethylphenyl)-sulfamoyl]-benzoylamino}-2-hydroxymethyl-essigsäure
3-{2-[N-(4-Fluor-2-trifluormethylphenyl)-sulfamoyl]-benzoylamino}-3-carboxy-propionsäure
3-{2-[N-(4-Fluor-3-trifluormethylphenyl)-sulfamoyl]-benzoylamino}-3-carboxy-propionsäure

4. Diagnostische Mittel enthaltend mindestens eine Verbindung der allgemeinen Formel I, gegebenenfalls mit den in der Galenik üblichen Zusätzen.

5. Verwendung von Verbindungen der allgemeinen Formel I als NMR-Diagnostika.

6. Verwendung von Verbindungen der allgemeinen Formel I zur in-vivo PH-Wert-Messung.

7. Verfahren zur Herstellung von Fluorbenzolsulfonamiden der allgemeinen Formel I

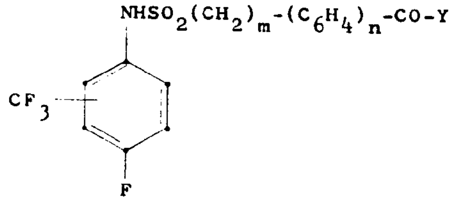

(I),

worin
m für die Ziffern 0, 1, 2, 3 oder 4,
n für die Ziffern 0 oder 1 und
Y für den Rest einer Aminocarbon- oder Aminosulfonsäure
stehen mit der Maßgabe, daß m und n nicht gleichzeitig für die Ziffer 0 stehen sollen und gewünschtenfalls die Säuregruppen in Form ihrer Amide oder in Form von Salzen mit organischen oder anorganischen Basen vorliegen,
dadurch gekennzeichnet, daß man in an sich bekannter Weise Verbindungen der allgemeinen Formel II

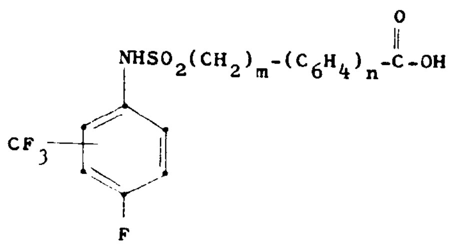

(II),

- gegebenenfalls in aktivierter Form -
mit Verbindungen der allgemeinen Formel III

H-Y' (III),

worin
Y' für den Rest einer gegebenenfalls geschützten Aminocarbon- oder Aminosulfonsäure oder für den Rest eines Aminocarbon- oder Aminosulfonsäureamids, worin gegebenenfalls vorhandene Hydroxygruppen gegebenenfalls geschützt sind,
umsetzt,
anschließend die Schutzgruppen entfernt, gewünschtenfalls die Säuregruppen mit organischen oder anorganischen Basen in die entsprechenden Salze überführt oder gegebenenfalls nach Aktivierung der in Y enthaltenen Säuregruppen, gewünschtenfalls mit einem Amin der allgemeinen Formel IV

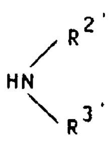

(IV),

worin
$R^{2'}$ und $R^{3'}$ die für $R^2$ und $R^3$ angegebene Bedeutung haben, wobei darin gegebenenfalls enthaltene Hydroxygruppen gegebenenfalls geschützt sind,
umsetzt und gegebenenfalls vorhandene Schutzgruppen entfernt.

8. Verfahren zur Herstellung der diagnostischen Mittel gemäß Anspruch 4, dadurch gekennzeichnet, daß

man die in Wasser oder physiologischer Salzlösung gelöste oder suspendierte Benzolverbindung, gegebenenfalls mit den in der Galenik üblichen Zusätzen in eine für die enterale oder parenterale Applikation geeignete Form bringt.

## Claims

1. Fluorobenzenesulphonamides of the general formula I

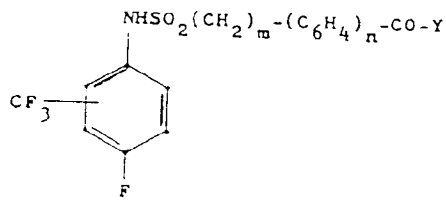

(I)

wherein

m represents the number 0, 1, 2, 3 or 4,

n represents the number 0 or 1 and

Y represents the radical of an aminocarboxylic or aminosulphonic acid,

with the proviso that m and n are not simultaneously to represent the number 0, and, if desired, the acid groups are in the form of their amides or in the form of salts with organic or inorganic bases.

2. Compounds according to claim 1, characterised in that Y represents the radicals

$-NH-CH_2COR^1$

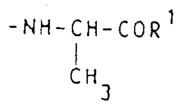

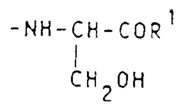

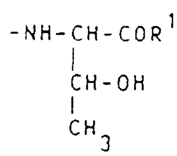

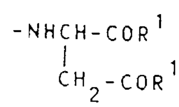

$-NH-CH_2-CH_2-COR^1$

$-N(CH_2COR^1)_2$

$-NH-(CH_2)_m-(C_6H_4)_n-SO_2R^1$

wherein $R^1$ represents a hydroxy group or a

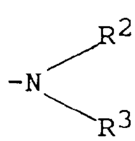

radical,

wherein

each of $R^2$ and $R^3$, independently of the other, represents a hydrogen atom, a linear or branched, saturated or unsaturated alkyl group having from 1 to 16 carbon atoms that is optionally substituted by from 1 to 5 hydroxy or $C_1$-$C_4$-alkoxy groups, or an aryl or aralkyl group, or

$R^2$ and $R^3$, together with the nitrogen atom, represent a saturated or unsaturated five- or six-membered ring optionally containing a further nitrogen, oxygen or sulphur atom or a carbonyl group.

3. 2-{2-[N-(4-fluoro-2-trifluoromethylphenyl)-sulphamoyl]-acetylamino}-acetic acid
2-{2-[N-(4-fluoro-3-trifluoromethylphenyl)-sulphamoyl]-acetylamino}-acetic acid
2-{2-[N-(4-fluoro-2-trifluoromethylphenyl)-sulphamoyl]-acetylamino}-2-hydroxymethylacetic acid
2-{2-[N-(4-fluoro-2-trifluoromethylphenyl)-sulphamoyl]-acetylamino}-2-hydroxymethylacetic acid
3-{2-[N-(4-fluoro-2-trifluoromethylphenyl)-sulphamoyl]-acetylamino}-3-carboxypropionic acid
3-{2-[N-(4-fluoro-3-trifluoromethylphenyl)-sulphamoyl]-acetylamino}-3-carboxypropionic acid
2-{4-[N-(4-fluoro-2-trifluoromethylphenyl)-sulphamoyl]-benzoylamino}-acetic acid
2-{4-[N-(4-fluoro-2-trifluoromethylphenyl)-sulphamoyl]-benzoylamino}-acetic acid
2-{4-[N-(4-fluoro-3-trifluoromethylphenyl)-sulphamoyl]-benzoylamino}-2-hydroxymethylacetic acid
2-{4-[N-(4-fluoro-2-trifluoromethylphenyl)-sulphamoyl]-benzoylamino}-2-hydroxymethylacetic acid
2-{4-[N-(4-fluoro-3-trifluoromethylphenyl)-sulphamoyl]-benzoylamino}-3-carboxypropionic acid
3-{4-[N-(4-fluoro-3-trifluoromethylphenyl)-sulphamoyl]-benzoylamino}-3-carboxypropionic acid
2-{2-[N-(4-fluoro-2-trifluoromethylphenyl)-sulphamoyl]-benzoylamino}-acetic acid
2-{2-[N-(4-fluoro-3-trifluoromethylphenyl)-sulphamoyl]-benzoylamino}-acetic acid
2-{2-[N-(4-fluoro-2-trifluoromethylphenyl)-sulphamoyl]-benzoylamino}-2-hydroxymethylacetic acid
2-{2-[N-(4-fluoro-2-trifluoromethylphenyl)-sulphamoyl]-benzoylamino}2-hydroxymethylacetic acid
3-{2-[N-(4-fluoro-2-trifluoromethylphenyl)-sulphamoyl]-benzoylamino}-3-carboxypropionic acid
3-{2-[N-(4-fluoro-3-trifluoromethylphenyl)-sulphamoyl]-benzoylamino}-3-carboxy propionic acid.

4. Diagnostic agents containing at least one compound of the general formula I, optionally together with the adjuvants customary in galenical pharmacy.

5. Use of compounds of the general formula I as NMR diagnostic agents.

6. Use of compounds of the general formula I for in vivo pH measurement.

7. Process for the preparation of fluorobenzene-sulphonamides of the general formula I

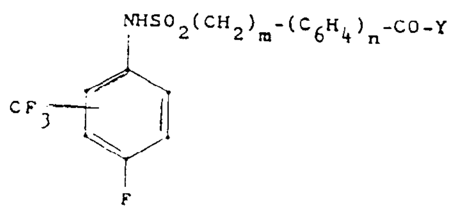

(I)

wherein
m represents the number 0, 1, 2, 3 or 4,
n represents the number 0 or 1 and
Y represents the radical of an aminocarboxylic or aminosulphonic acid,
with the proviso that m and n are not simultaneously to represent the number 0, and, if desired, the acid groups are in the form of their amides or in the form of salts with organic or inorganic bases,
characterised in that compounds of the general formula II

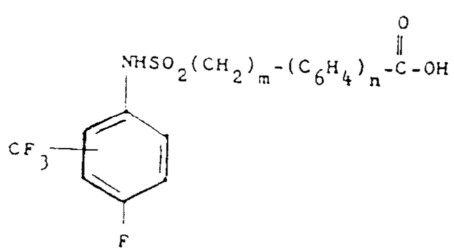

(II)

- optionally in activated form -
are reacted in a manner known per se with compounds of the general formula III

H-Y′ (III)

wherein

Y' represents the radical of an optionally protected aminocarboxylic or aminosulphonic acid or the radical of an aminocarboxylic or aminosulphonic acid amide, wherein any hydroxy groups present are optionally protected,

protecting groups are then removed, the acid groups are, if desired, converted into the corresponding salts with organic or inorganic bases or, optionally after activating the acid groups contained in Y, they are, if desired, reacted with an amine of the general formula IV

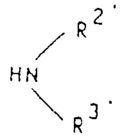

(IV)

wherein

$R^{2'}$ and $R^{3'}$ are as defined for $R^2$ and $R^3$, any hydroxy groups contained therein optionally being protected,

and any protecting groups present are removed.

8. Process for the preparation of the diagnostic agents according to claim 4, characterised in that the benzene compound dissolved or suspended in water or physiological saline solution is brought into a form suitable for enteral or parenteral administration, optionally together with the adjuvants customary in galenical pharmacy.

## Revendications

1. Fluorobenzènesulfonamides de formule générale I :

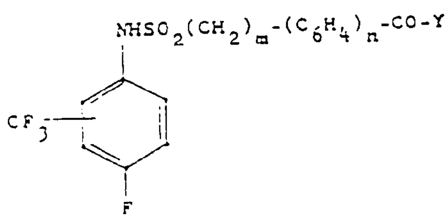

(I)

dans laquelle

m représente les nombres 0, 1, 2, 3 ou 4,

n représente les nombres 0 ou 1, et

Y représente le radical d'un acide aminocarboxylique ou aminosulfonique,

à la condition que m et n ne correspondent pas simultanément au nombre 0 et que si cela est souhaitable, les groupes acides peuvent être présents sous forme de leurs amides ou sous forme de sels de bases organiques ou minérales.

2. Composés selon la revendication 1, caractérisés en ce que Y représente les radicaux :

$-NH-CH_2COR^1$

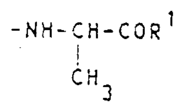

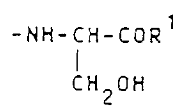

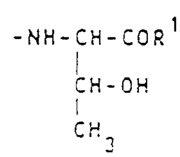

$$-NHCH-COR^1$$
$$|$$
$$CH_2-COR^1$$

$-NH-CH_2-CH_2-COR^1$

$-N(CH_2COR^1)_2$

$-NH-(CH_2)_m-(C_6H_4)_n-SO_2R^1$

avec $R^1$ ayant la signification d'un groupe hydroxy ou d'un radical

$R^2$
/
$-N$
\
$R^3$

où

$R^2$ et $R^3$ indépendamment l'un de l'autre représentent les atome d'hydrogène, groupe alkyle linéaire ou ramifié, saturé ou insaturé, éventuellement substitué par 1 à 5 groupes hydroxy ou alcoxy en $C_1$-$C_4$, comportant de 1 à 16 atomes de carbone, ou un groupe aryle ou aralkyle, ou

$R^2$ et $R^3$ ensemble avec l'atome d'azote signifient un cycle à cinq ou six chaînons saturé ou insaturé contenant éventuellement un autre atome d'azote, d'oxygène, de soufre ou un groupe carbonyle.

**3.** Acide 2-{2-[N-(4-fluoro-2-trifluorométhylphényl)-sulfamoyl]-acétylamino}-acétique
Acide 2-{2-[N-(4-fluoro-3-trifluorométhylphényl)-sulfamoyl]-acétylamino}-acétique
Acide 2-{2-[N-(4-fluoro-2-trifluorométhylphényl)-sulfamoyl]-acétylamino}-2-hydroxyméthyl-acetique
Acide 2-{2-[N-(4-fluoro-3-trifluorométhylphényl)-sulfamoyl]-acétylamino}-2-hydroxyméthyl-acétique
Acide 3-{2-[N-(4-fluoro-2-trifluorométhylphényl)-sulfamoyl]-acétylamino}-3-carboxy-propionique
Acide 3-{2-[N-(4-fluoro-3-trifluorométhylphényl)-sulfamoyl]-acétylamino}-3-carboxy-propionique
Acide 2-{4-[N-(4-fluoro-2-trifluorométhylphényl)-sulfamoyl]-benzoylamino}-acétique
Acide 2-{4-[N-(4-fluoro-3-trifluorométhylphényl)-sulfamoyl]-benzoylamino}-acétique
Acide 2-{4-[N-(4-fluoro-2-trifluorométhylphényl)-sulfamoyl]-benzoylamino}-2-hydroxyméthyl-acétique
Acide 2-{4-[N-(4-fluoro-3-trifluorométhylphényl)-sulfamoyl]-benzoylamino}-2-hydroxyméthyl-acétique
Acide 3-{4-[N-(4-fluoro-2-trifluorométhylphényl)-sulfamoyl]-benzoylamino}-3-carboxy-propionique
Acide 3-{4-[N-(4-fluoro-3-trifluorométhylphényl)-sulfamoyl]-benzoylamino}-3-carboxy-propionique
Acide 2-{2-[N-(4-fluoro-2-trifluorométhylphényl)-sulfamoyl]-benzoylamino}-acétique
Acide 2-{2-[N-(4-fluoro-3-trifluorométhylphényl)-sulfamoyl]-benzoylamino}-acétique
Acide 2-{2-[N-(4-fluoro-2-trifluorométhylphényl)-sulfamoyl]-benzoylamino}-2-hydroxyméthyl-acétique
Acide 2-{2-[N-(4-fluoro-3-trifluorométhylphényl)-sulfamoyl]-benzoylamino}-2-hydroxyméthyl-acétique
Acide 3-{2-[N-(4-fluoro-2-trifluorométhylphényl)-sulfamoyl]-benzoylamino}-3-carboxy-propionique
Acide 3-{2-[N-(4-fluoro-3-trifluorométhylphényl)-sulfamoyl]-benzoylamino}-3-carboxy-propionique

**4.** Agent diagnostique contenant au moins un composé de formule générale I, éventuellement avec des adjuvants usuels en galénique.

**5.** Utilisation des composés de formule générale I en tant que produits de diagnostic pour RMN.

**6.** Utilisation des composés de formule générale I pour la mesure in vivo de la valeur du pH.

**7.** Procédé pour la préparation de fluorobenzènesulfonamides de formule générale I :

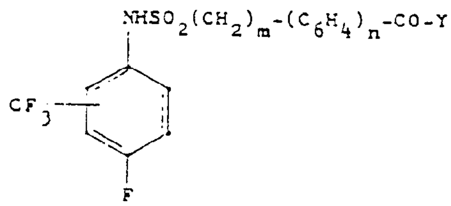

(I)

dans laquelle

m représente les nombres 0, 1, 2, 3 ou 4,

n représente les nombres 0 ou 1, et

Y représente le radical d'un acide aminocarboxylique ou aminosulfonique,

à la condition que m et n ne représentent pas simultanément le nombre 0 et si on le désire, les groupes acides peuvent être présents sous forme de leurs amides ou leurs sels avec des bases organiques ou minérales,

caractérisé en ce qu'on fait réagir de façon connue en soi des composés de formule générale II :

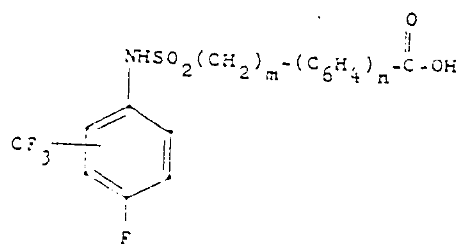

(II)

-éventuellement sous forme activée-

avec des composés de formule générale III :

H-Y' (III)

dans laquelle

Y' représente le radical d'un acide aminocarboxylique ou aminosulfonique éventuellement protégé ou le radical d'un amide d'acide aminocarboxylique ou aminosulfonique où les groupes hydroxy éventuellement présents sont éventuellement protégés,

on élimine ensuite les groupes protecteurs, si on le désire, on transforme les groupes acides avec des bases organiques ou minérales en sels correspondants ou éventuellement, après activation des groupes acides contenus dans Y, si on le désire, avec une amine de formule générale IV :

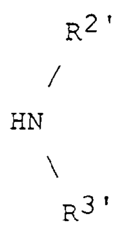

(IV)

dans laquelle

$R^{2'}$ et $R^{3'}$ ont les significations données pour $R^2$ et $R^3$, les groupes hydroxy éventuellement contenus étant éventuellement protégés,

et qu'on élimine les groupes protecteurs éventuellement présents.

**8.** Procédé pour la préparation d'agents diagnostiques selon la revendication 4, caractérisé en ce que le composé benzénique dissous ou suspendu dans l'eau ou dans une solution saline physiologique, éventuellement avec des adjuvants usuels en galénique, est mis en forme appropriée pour application entérale ou parentérale.